# EUROPEAN PATENT APPLICATION

(11) **EP 3 235 512 A1**
(43) Date of publication of application: **25.10.2017**
(21) Application number: 15869318.4
(22) Date of filing: 15.12.2015
(51) Int. Cl.: A61K 47/42, A61K 9/16

(54) **METHOD USING POLYETHYLENE GLYCOL TO PREPARE FIBROIN NANO/MICROSPHERES, AND APPLICATION OF METHOD IN CONTROLLED DRUG RELEASE**

(30) Priority: 16.12.2014 CN 201410777934; 13.12.2015 CN 201510918623
(71) Applicant: Simatech Incorporation, Suzhou, Jiangsu 215123 (CN)
(72) Inventor: WU, Jianbing, Suzhou Jiangsu 215000 (CN); LIU, Jian, Suzhou Jiangsu 215000 (CN)
(74) Representative: Bianchetti Bracco Minoja S.r.l.
(86) International application number: PCT/CN2015/097464
(87) International publication number: WO 2016/095811

(57) **Abstract**

A method using polyethylene glycol to prepare fibroin nano/microspheres. A fibroin solution having a mass percentage of 1-30% and a polyethylene glycol solution having a mass percentage of 10-60% are first placed in a 4-60°C environment for 30 minutes, the fibroin protein solution and the polyethylene glycol solution are then mixed, and fibroin nano/microspheres are produced via incubation and centrifugal washing.

## Description

### Technical field

The present invention belongs to biomedicinal or pharmaceutical field, and particularly relates to the method for preparation of fibroin nano/microspheres using polyethylene glycol and its application in controlled release of drugs.

### Background art

Fibroin is a natural polymer fibrin extracted from natural silk and accounts for about 70%-80% of the silk. Fibroin has excellent physicochemical properties, is non-toxic, nonirritant, biocompatible and biodegradable, can greatly satisfy the demands of biomaterials. Meanwhile, fibroin has excellent processability, may be prepared into membranes, gels, microspheres, porous stents, etc, and can be used as drug carriers.

Chinese patent application No. 201310203722.2 discloses a preparing method for an injectable *in situ* gelatin of fibroin, in which gels are formed by mixing the fibroin aqueous solution with the solution of poly-ethylene glycol or propylene glycol, followed by solidification. Since the particle size of the gel granules is too small to be suitable for drug-loading, there is a need to develop a simple and feasible method for preparation of fibroin nano/microspheres intended to be used as drug carriers.

Currently, the preparative methods for fibroin nano/microspheres mainly include emulsion-organic solvent evaporation/extraction, phase separation, self-assembly, rapid expansion of supercritical fluid, method of preparing liposome template, microfluidic method and spray drying, and the like. Emulsification, self-assembly, and liposome template method all need addition of organic solvents, while the residue of organic solvents can directly limit the use of fibroin nano/microspheres in clinic. Although rapid expansion of supercritical fluid, microfluidic method and spray drying are relatively simple, the devices and the preparative conditions are relatively complicated. Though the preparative procedures of phase separation are relatively simple, safe and non-toxic, the operation process is complicated.

On the other hand, as drug carriers, fibroin has obvious advantages: firstly, its source is plenty, and the cost is low; secondly, as natural polymers, fibroin has good biodegradability and high biosafety; thirdly, fibroin is characterized by various crystallization modes, and the conformation construction can be regulated for the purpose of controlling the release ratio of drugs, allowing the release of drugs in target regions, thereby improving the therapeutic effect of drugs. Accordingly, by preparing fibroin having excellent biocompatibility into micro-spheres/particles, a synergistic action will be obtained, wherein the micro-spheres/particles of fibroin not only have biocompatibility incomparable to other materials, but also have higher bioavailability and biosafety.

At present, the methods for preparation of drug-loaded nano-/microparticles/spheres of fibroin mainly include emulsion-organic solvent evaporation/extraction, phase separation, self-assembly, rapid expansion of supercritical fluid, method of preparing liposome template, microfluidic method and spray drying, and the like, wherein:
For emulsion-organic solvent evaporation/extraction, Thanonchat discloses a method comprising dispersing fibroin solution in organic solvent ethyl acetate and then adding into an oil phase containing surfactants (such as Span 80) with stirring or sonication, thereby forming a (W/O type) emulsion, to which chemical crosslinking agents (glutaraldehyde) are added for solidification. This method utilizes the feature that the amino of fibroin can readily react with the aldehyde group of other compounds via condensation polymerization, thus generating nano/microparticles of fibroin by cross-linking. Nano/microparticles of fibroin in emulsion can be separated by evaporation of or extraction with organic solvent.

For phase separation, Wang discloses mixing the fibroin solution and the PVA (polyvinyl alcohol) solution directly and, after sonication or stirring, drying the blending solution in an oven at 60 °C, then dissolving the dried residue again and subjecting to centrifugal washing to prepare nano/microparticles of fibroin. The particle size of fibroin nano/microparticles can be regulated by changing the concentration of fibroin solution and the power of sonication.

For self-assembly, Cao discloses mixing the fibroin solution and the ethanol solution directly and, after stirring at room temperature for 2 min and freezing in fridge (-5 °C--40 °C) for 24 h, thawing at room temperature and centrifugal washing to afford nano/microparticles of fibroin. The particle size of fibroin nano/microparticles can be regulated by changing the concentration of fibroin solution as well as the volume ratio of fibroin solution to ethanol.

For rapid expansion of supercritical fluid, Wenk discloses dissolving the solution of drug-loaded fibroin in superfluid and, in a short period of time, spouting the superfluid from a nozzle of certain pore size using a frequency-generating vibration system. Under the conditions of high voltage, the fibroin solution rapidly generates crystal nucleus and the crystal nucleus expand quickly in a short time. Liquid droplets are collected by liquid nitrogen collecting unit and quickly frozen to afford drug-loaded fibroin microspheres. The particle size of fibroin nano/microparticles can be directly regulated by the pore size of nozzle.

For method of preparing liposome template, Wang discloses dissolving DOPC (dioleoylphosphatidyl choline) in organic solvent chloroform and drying the resultant solution under nitrogen to form membranes, to which a certain volume of fibroin solution is added. After dilution with deionized water, the mixture is frozen in liquid nitrogen for 15 min and then thawed at 37°C for 15 min, and the thawed solution is poured in a beaker with quick stirring. The solution is then lyophilized and stored in fridge at 4 °C. By treatment with methanol and sodium chloride solution, degradation of liposomes takes place and induces the structural change of fibroin, thus fibroin nano/microparticles are generated.

For microfluidic method, Mitropoulos discloses using the principle of phase separation, the fibroin solution and the PVA (polyvinyl alcohol) solution are isolated by a microfluidic device, and the particle size of fibroin nano/microparticles can be regulated by controlling the flow rate for mixing of solutions, and the particle size of obtained microspheres is relatively uniform.

For spray drying, Hino discloses directly spraying the fibroin solution into hot air with atomization device, and the fibroin nano/microparticles are generated during the drying process.

The above mentioned methods for preparation of fibroin nano/microparticles still have shortcomings and thus limit the drug loading and applications in later stage. For example, emulsion-organic solvent evaporation/extraction, self-assembly, and liposome template method all need addition of organic solvents, while the residue of organic solvents can affect the stability of small molecular drugs and macromolecular protein drugs, thus directly limit the use of fibroin nano/microparticles in clinic. Although the methods such as rapid expansion of supercritical fluid, microfluidic method and spray drying are relatively simple, the devices and the preparative conditions are relatively complicated and the drug-loading ratio is low, thereby the cost is high and the industrialized applications of these methods in later stage are limited. Consequently, there is an urgent need for developing a method of preparing drug-loaded fibroin nano/microspheres which have good controlled release effect on drugs and no residue of organic solvent, and said method should have advantages of simple operation, low cost, and good embedding effect.

### Summary of the invention

In view of drawbacks presented by above fibroin nano/microspheres and in controlled release of drugs, the object of present invention is intended to overcome disadvantages of preparative process of drug-loaded fibroin nano/microparticles, such as residue of organic solvents, multifarious process, time-consuming, high cost, not suitable for the industrialized production and clinical applications, and provide a preparing method with advantages of high efficiency and safety, fast and simple, good resource utilization, low production cost, good biocompatibility, having values for industrialized production and clinical applications. This method not only realizes controlled release of drugs by regulating the particle size and the secondary crystal structures of fibroin granules, but also resolves the technical problem that a part of clinical drugs are unable to be fixed in fibroin nano/microparticles by physical embedding method.

The present invention provides a method for preparation of fibroin nano/microspheres using polyethylene glycol, comprising the following steps:
1-30 wt% fibroin solution and 10-60 wt% polyethylene glycol solution are placed at a temperature of 4-60 °C for 30 min, and then the fibroin solution and the polyethylene glycol solution are uniformly mixed at a certain ratio, and after incubation for a certain period of time, the mixture is subjected to centrifugal washing to afford suspension of fibroin nano/microspheres.

On the other hand, the present invention provides use of above method for preparation of fibroin nano/microspheres using polyethylene glycol, wherein said method is used in the preparation of drug-loaded fibroin nano/microparticles and in controlled release of drugs, comprising the following steps:
dissolving the drug in 10-60 wt% polyethylene glycol solution or dissolving the drug in 1-30 wt% fibroin solution, and then the fibroin solution and the polyethylene glycol solution are uniformly mixed to produce a blending solution, and after incubation the blending solution for a certain period of time and centrifugal washing, drug-loaded fibroin nano/microspheres used for controlled release of drugs are obtained.

In another embodiment of the present invention, when said drugs are hydrophobic, said hydrophobic drugs are dissolved in the solution of polyethylene glycol before mixing with the fibroin solution to prepare the blending solution; and for other drugs than hydrophobic drugs, said other drugs are firstly dissolved in the solution of fibroin before mixing with the solution of polyethylene glycol to prepare the blending solution.

In another embodiment of the present invention, said hydrophobic drugs include but are not limited by curcumin, while said other drugs include hydrophilic drug adriamycin, polypeptide drug octreotide, protein drug bovine serum albumin, macromolecular hydrophilic model drug glucan or fluorescent labeled CdTe quantum dot.

In another embodiment of the present invention, the mass ratio of fibroin contained in said fibroin solution to the drug is in the range of 1000/1-1/10.

In another embodiment of the present invention, the molecular weight of polyethylene glycol is in the range of 2000-20000.

In another embodiment of the present invention, when the molecular weight of polyethylene glycol is 4000, 6000, its mass percent is 30-60%; when the molecular weight of polyethylene glycol is 10000, its mass percent is 20-50%; when the molecular weight of polyethylene glycol is 20000, its mass percent is 20-40%.

In another embodiment of the present invention, the pH value of said fibroin solution is in the range of 3.0-11.0.

In another embodiment of the present invention, the salt ions concentration for treatment of fibroin solution is in the range of 1 mol/L to 0.01 mol/L.

In another embodiment of the present invention, the volume ratio of said fibroin solution to said polyethylene solution is in the range of 5:1-1:10.

In another embodiment of the present invention, said blending solution is incubated at a temperature from room temperature to 60 °C for 0.5-24 h.

In another embodiment of the present invention, the dilution range of said fibroin solution is 5-20 wt%.

In another embodiment of the present invention, the preparing procedure of said fibroin is as follows: the degummed, boiled-off silk is soaked in a 9.3 M solution of LiBr and placed in an oven at 60 °C for 4 h, and after dissolving, the fibroin solution is obtained; said fibroin solution is poured into a dialysis bag and dialyzed against deionized water for 3 days; after completion of dialysis, the fibroin solution is centrifugated to remove insoluble impurities, and then poured into a dialysis bag and dialyzed against 15 wt% solution of polyethylene glycol having a molecular weight of 20000 for 24 h, to obtain the concentrated solution of fibroin. Alternatively, the concentrated solution of fibroin can be obtained by dissolving lyophilized silk powder in water.

Based on above embodiments, the present invention at least has the following advantages:
1. In the present invention, the pharmaceutically acceptable adjuvant polyethylene glycol is mixed with the solution of fibroin to prepare fibroin nano/microspheres, alternatively, the medicinal adjuvant polyethylene glycol is mixed with the solution of fibroin to prepare fibroin particles. The preparative process doesn't need complicated devices, and the operation process is simple and feasible, together with short time-consuming and low cost as well as without addition of organic solvents. The biosafety of the prepared fibroin particles is high, and the particles can be directly used in clinic, attaining optimal utilization of resources;
2. The method according to the present invention is intended to directly mix, incubate, and quickly prepare fibroin particles, said method doesn't need complex devices, doesn't need to dry to form membranes and dissolve again, and thus reduces the operation process, shortens the preparative time, and benefits for industrialized production;
3. The method according to the present invention is intended to prepare fibroin nano/microparticles at ambient temperature, and thus doesn't need to freeze in fridge or dry in an oven, that not only saves energy and reduces the production cost, but also amenable to the loading and stabilization of bioactive molecules;
4. The particle size of the fibroin granules prepared according to the present invention can be regulated by changing the relative molecular weight and the concentration of polyethylene glycol and the concentration of fibroin, that create conditions for controlled release and loading of drugs;
5. The fibroin particles prepared according to the present invention can be used to embed sustained-release drugs which can be selected from a wide range, including hydrophilic and hydrophobic small molecular drugs, polypeptide drugs, protein drugs, etc., in which hydrophobic neutral drugs can bond to specific hydrophobic region of fibroin molecules by hydrophobic interaction and be embedded in fibroin particles, thereby the drug-loading ratio of hydrophobic neutral drugs is improved.

The above illustration is merely summary of technical solutions according to the present invention. In order to better understand the technical means of the invention and practice the invention according to the disclosure of the specification, the present invention is further described in the following examples.

### Description of Figures

Figure 1 shows the scanning electron micrograph of fibroin nano/microparticles prepared in example 1-4 of the present invention;
Figure 2 shows the particle size distribution pattern of fibroin nano/microspheres prepared by mixing solutions of PEG having different molecular weight with 8wt% fibroin solution in example 1-4 of the present invention;
Figure 3 shows the scanning electron micrograph of fibroin nano/microparticles prepared in example 1-6 of the present invention;
Figure 4 is a chart of the particle size distribution of fibroin nano/microparticles prepared in example 2 of the present invention *vs*. the viscosity of polyethylene glycol solution;
Figure 5 shows the scanning electron micrograph of fibroin nano/microparticles prepared in example 3 of the present invention;
Figure 6 is a chart of the particle size distribution of fibroin nano/microparticles prepared in example 3 of the present invention *vs*. the viscosity of polyethylene glycol solution;
Figure 7 shows the scanning electron micrograph of fibroin nano/microparticles prepared in example 4 of the present invention;
Figure 8 shows the particle size distribution pattern of fibroin nano/microparticles prepared in example 4 of the present invention;
Figure 9 shows the scanning electron micrograph of fibroin nano/microparticles prepared in example 5 of the present invention;
Figure 10 shows the scanning electron micrograph of fibroin nano/microparticles prepared in example 6 of the present invention;
Figure 11 shows the scanning electron micrograph of fibroin nano/microparticles prepared in example 6 of the present invention;
Figure 12 shows the scanning electron micrograph of fibroin nano/microparticles prepared in example 7 of the present invention;
Figure 13 shows the particle size distribution pattern of fibroin nano/microparticles prepared in example 7 of the present invention;
Figure 14 shows the scanning electron micrograph of fibroin nano/microparticles prepared in example 8 of the present invention;
Figure 15 shows the surface morphology of single microsphere of fibroin prepared in example 8 of the present invention;
Figure 16 shows the cross section morphology of single microsphere of fibroin prepared in example 8 of the present invention;
Figure 17 shows the particle size distribution pattern of fibroin nano/microparticles prepared in example 8 of the present invention;
Figure 18 shows the zete potential diagram of fibroin nano/microparticles prepared in example 8 of the present invention;
Figure 19 shows the yield map of fibroin nano/microparticles prepared in example 8 of the present invention;
Figure 20 shows the IR spectrum of fibroin nano/microparticles prepared in example 9 of the present invention;
Figure 21 shows the secondary structure content of fibroin nano/microparticles prepared in example 9 of the present invention and the control sample;
Figure 22 shows the IR spectrum of fibroin nano/microparticles prepared in example 10 of the present invention;
Figure 23 shows the secondary structure content of fibroin nano/microparticles prepared in example 10 of the present invention and the control sample;
Figure 24 shows the IR spectrum of fibroin nano/microparticles prepared in example 11 of the present invention;
Figure 25 shows the secondary structure content of fibroin nano/microparticles prepared in example 11 of the present invention and the control sample;
Figure 26 shows the IR spectrum of fibroin nanoparticles (supernatant) prepared in example 12 of the present invention;
Figure 27 shows the IR spectrum of fibroin microparticles (standing twice) prepared in example 12 of the present invention;
Figure 28 shows the IR spectrum of fibroin nanoparticles (standing once) prepared in example 12 of the present invention;
Figure 29 shows the confocal laser scanning micrograph of nano/microparticles of curcumin-fibroin prepared in example 13 of the present invention;
Figure 30 shows the confocal laser scanning micrograph of nano/microparticles of TMR-dextran-fibroin prepared in example 14 of the present invention;
Figure 31 shows the fluorescence micrograph of nano/microparticles of CdTe-fibroin prepared in example 15 of the present invention;
Figure 32 shows the scanning electron micrograph of nano/microparticles of curcumin-fibroin prepared in example 16 of the present invention;
Figure 33 shows the confocal laser scanning micrograph of nano/microparticles of curcumin-fibroin prepared in example 16 of the present invention;
Figure 34 shows the scanning electron micrograph of nano/microparticles of doxorubicin hydrochloride-fibroin prepared in example 17 of the present invention;
Figure 35 shows the confocal laser scanning micrograph of nano/microparticles of doxorubicin hydrochloride-fibroin prepared in example 17 of the present invention;
Figure 36 shows the cumulative release ratio chart of nano/microparticles of curcumin-fibroin prepared in example 18 of the present invention;
Figure 37 shows the cumulative release ratio chart of nano/microparticles of TMR-dextran-fibroin prepared in example 19 of the present invention;
Figure 38 shows the cumulative release ratio chart of nano/microparticles of CdTe-fibroin prepared in example 20 of the present invention;
Figure 39 shows the cumulative release ratio chart of nano/microparticles of curcumin-fibroin prepared in example 21 of the present invention;
Figure 40 shows the cumulative release ratio chart of nano/microparticles of doxorubicin hydrochloride-fibroin prepared in example 22 of the present invention;
Figure 41 shows the cumulative release ratio chart of nano/microparticles of doxorubicin hydrochloride-fibroin prepared in example 23 of the present invention;
Figure 42 shows the scanning electron micrograph of nano/microparticles of octreotide-fibroin prepared in example 24 of the present invention;
Figure 43 shows the scanning electron micrograph of nano/microparticles of TMR-bovine serum albumin-fibroin prepared in example 25 of the present invention;
Figure 44 shows the confocal laser scanning micrograph of nano/microparticles of TMR-bovine serum albumin-fibroin prepared in example 25 of the present invention;
Figure 45 shows the cumulative release ratio chart of nano/microparticles of TMR-bovine serum albumin-fibroin prepared in example 26 of the present invention;
Figure 46 shows the scanning electron micrograph of nanoparticles (supernatant) of doxorubicin hydrochloride-fibroin prepared in example 27 of the present invention;
Figure 47 shows the scanning electron micrograph of microparticles (sedimentation once) of doxorubicin hydrochloride-fibroin prepared in example 27 of the present invention;
Figure 48 shows the particle size distribution pattern of nanoparticles (supernatant) of doxorubicin hydrochloride-fibroin prepared in example 27 of the present invention;
Figure 49 shows the zete potential diagram of nanoparticles (supernatant) of doxorubicin hydrochloride-fibroin prepared in example 27 of the present invention;
Figure 50 shows the encapsulation rate diagram of nano/microparticles of doxorubicin hydrochloride-fibroin prepared in example 27 of the present invention;
Figure 51 shows the confocal laser scanning micrograph of microparticles (standing once) of doxorubicin hydrochloride-fibroin prepared in example 27 of the present invention;

### Embodiments

The technical solution in examples of the present invention will be clearly and integrally illustrated hereinafter, and obviously, the described examples are only a part but not all of the examples in the present invention, and these examples are not intended to limit the scope of the present invention. On the basis of examples of the present invention, all other examples acquired by those skilled in the art without any creative works are within the protection scope of the present invention.

In order to maintain the stability and molecular activity of drug delivery, for the method for preparation of drug-loaded fibroin nano/microparticles used for controlled release of drugs according to the present invention, it is necessary to avoid the use of organic solvents and severe particles/spheres forming conditions in the preparation process. Comparing with current methods of preparing nano/micropartiles by phase separation, the particle size and the morphology of microspheres prepared by phase separation can be regulated by changing the molecular weight of PVA (polyvinyl alcohol) as well as the weight ratio of PVA to fibroin. Though the preparative procedure of phase separation is relatively simple, safe and non-toxic, the blending solution of fibroin and PVA needs to be subjected to membrane forming, dissolving, washing and the like, and thus the process is relatively complicated.

The method according to the present invention is different from current phase separation method in principles, operation process, and drug applicability. The differences in principle mainly lie in the following: in phase separation, PVA solution and fibroin solution are mixed, and fibroin molecules are thoroughly dispersed in PVA solution by ultrasounding or stirring, then the dispersed solution is placed in an oven for drying and forming membrane and, after dissolving and washing, particles were obtained. On the other hand, the present invention uses the emulsion polymerization method, and fibroin solution is directly added in polyethylene glycol solution. With aid of its hydrophilicity, polyethylene glycol can rapidly absorb water molecules on surface of fibroin, and promote the hydrophobic regions of fibroin to approach each other, inducing aggregation among molecules to form self-assembled microspheres. The differences in operation process lies in the following: the phase separation method requires that the blending solution be placed in an oven at 60 °C for drying and forming membrane, followed by dissolving and washing, while the present invention only needs incubation at room temperature without forming a membrane. The differences in drug applicability lies in the following: fibroin particles prepared by phase separation have a low drug-loading ratio for hydrophobic neutral drugs, while polyethylene glycol used in the method of the present invention is an amphiphilic medicinal adjuvant, and hydrophobic neutral drugs can be dissolved in polyethylene glycol solution, then mixed and incubated with fibroin to form microspheres, thus the drug-loading ratio of fibroin particles is higher.

As far as controlled release of drugs, the drug-loaded fibrion nano/microparticles according to the present invention realize quantitive and zero-order or first-order release of drugs, and thus keep the blood level constant. By selecting small molecular drugs such as curcumin and adriamycin hydrochloride, large molecular drugs such as TMR-glucan, polypeptide and protein drugs such as octreotide and TMR-bovine serum albumin and the like and loading them on fibroin nano/microparticles by physical embedding, the present invention provides possibilities for clinical application of fibroin nano/microparticles as drug carrier in later stage.

### Example 1

### 1-1. Preparation of fibroin solution

25 g of degummed, boiled-off silk was weighed and soaked in 9.3 M solution of LiBr (100 ml), and then placed in an oven at 60 °C for 4 h. The resulting mixture was stirred at one hour intervals till thorough dissolution, to obtain 20% (W/V) fibroin solution. The fibroin solution was poured into a Slide-a-lyzer dialysis bag with a molecular cut off of 3500, and the bag was transferred to a dialysis tank charged with deionized water and kept for 3 days. During dialysis, the deionized water was exchanged 10 times. After completion of dialysis, the fibroin solution was centrifuged under 9000 rpm/min at 4 °C for 20 min, to remove insoluble impurities. To determine the concentration of fibroin solution, a certain amount of fibroin solution was taken out and weighed, and then dried in an oven at 60 °C to dryness and weighed. The ratio of the two weights (fibroin solution and dried material) represents the concentration (mass percent, wt%). In general, the concentration of obtained fibroin solution was about 7 wt%. For the concentration of fibroin solution, the prepared fibroin solution (100 ml) was transferred into the same type of Slide-a-lyzer dialysis bag, and the bag was placed in a 15 wt% solution of polyethylene glycol having a molecular weight of 20000 (800 ml) for 24 h successive dialysis, to obtain about 30wt% concentrated solution of fibroin, which was stored in a fridge at 4°C.

### 1-2. Preparation of polyethylene glycol-fibroin blending solution

The 30 wt% fibroin solution prepared in 1-1 was diluted to 1%-5wt%;
Polyethylene glycols having different molecular weight were prepared into solutions at different concentrations, and the concentration of polyethylene glycol having a molecular weight of less than 1000 was 40-100wt%; the concentration of polyethylene glycol having a molecular weight of 1000-6000 was 10-60wt%; the concentration of polyethylene glycol having a molecular weight of 10000 was 10-50wt%; and the concentration of polyethylene glycol having a molecular weight of 20000 was 10-40wt%;
Equal volume of 1-5 wt% fibroin solution was respectively taken out and added to same volume of polyethylene glycol solutions having different molecular weights and concentrations, and then gently agitated up and down with a pipette to mix the blending solution uniformly;
The polyethylene glycol-fibroin blending solution was incubated at room temperature for 30 min;
After incubation, the state of blending solution was observed and recorded.

### 1-3. Preparation of polyethylene glycol-fibroin blending solution

The 30 wt% fibroin solution prepared in 1-1 was diluted to 6%-30wt%;
Polyethylene glycols having different molecular weight were prepared into solutions at different concentrations, and the concentration of polyethylene glycol having a molecular weight of 2000-6000 was 10-60wt%; the concentration of polyethylene glycol having a molecular weight of 10000 was 10-50wt%; and the concentration of polyethylene glycol having a molecular weight of 20000 was 10-40wt%;
Equal volume of 6-30 wt% fibroin solution was respectively taken out and added to same volume of polyethylene glycol solutions having different molecular weights and concentrations, and then gently agitated up and down with a pipette to mix the blending solution uniformly;
The polyethylene glycol-fibroin blending solution was incubated at room temperature for 30 min;
After incubation, the state of blending solution was observed and recorded.

Experimental results of 1-1 and 1-2 were summarized in Table 1. From the recorded values, it can be seen that polyethylene glycols with high molecular weight readily mixed with the fibroin solution to form particles; the molecular weight and the concentration of polyethylene glycol as well as the concentration of fibroin are direct factors affecting the particle formation.

**Table 1. State of the blending solution 30 minutes after the mixing of fibroin solution and polyethylene glycol solution**

| PEG molecular weight (Da) | PEG concentration (wt%) | Fibroin concentration (wt%) | Change of solution state after blending for 30 minutes |
|---|---|---|---|
| 200 | 100 | 1-5 | 1wt % solution, 2-5wt % gel-forming |
| | 50 | 1-5 | 1-4 wt% solution, 5 wt% gel-forming |
| | 45 | 1-5 | |
| | 40 | 1-5 | |
| 300 | 100 | 1-5 | 1-5 wt% gel-forming |
| | 55 | 1-5 | 1-2 wt% solution, 3-5 wt% gel-forming |
| | 50 | 1-5 | 1-3 wt% solution, 4-5 wt% gel-forming |
| | 45 | 1-5 | |
| 400 | 100 | 1-5 | 1-5 wt% gel-forming |
| | 55 | 1-5 | 1 wt% solution, 2-5 wt% gel-forming |
| | 50 | 1-5 | |
| | 45 | 1-5 | |
| 600 | 100 | 1-5 | 1-5 wt% gel-forming |
| | 60 | 1-5 | 1-2 wt% solution, 3-5 wt% gel-forming |
| | 55 | 1-5 | 1-3 wt% solution, 4-5 wt% gel-forming |
| | 50 | 1-5 | 1-5 wt% solution |
| 1000 | 60 | 1-5 | 1 wt% solution, 2-5 wt% gel-forming |
| | 55 | 1-5 | 1-2 wt% solution, 3-5 wt% gel-forming |
| | 10 | 1-5 | 1-5 wt% solution |
| 1500 | 60 | 1-5 | 1 wt% solution, 2-5 wt% gel-forming |
| | 55 | 1-5 | 1-2 wt% solution, 3-5 wt% gel-forming |
| | 10 | 1-5 | 1-3 wt% solution, 4-5 wt% gel-forming |
| 2000 | 60 | 1-30 | 1 wt% solution, 2-30 wt% gel-forming |
| | 50 | 1-30 | 1-2 wt% solution, 3-30 wt% gel-forming |
| | 40 | 1-30 | 1-2 wt% solution, 3-30 wt% particle-forming |
| | 10 | 1-30 | 1-30 wt% solution |
| 4000 | 60 | 1-30 | 1-3 wt% solution, 4-30 wt% particle-forming |
| | 40 | 1-30 | 1-4 wt% solution, 5-30 wt% particle-forming |
| | 10 | 1-30 | 1-30 wt% solution |
| 6000 | 60 | 1-30 | 1-3 wt% solution, 4-30 wt% particle-forming |
| | 40 | 1-30 | 1-3 wt% solution, 4-30 wt% particle-forming |
| | 10 | 1-30 | 1-30 wt% solution |
| 10000 | 50 | 1-30 | 1-2 wt% solution, 3-30 wt% particle-forming |
| | 25 | 1-30 | 1-3 wt% solution, 4-30 wt% particle-forming |
| | 10 | 1-30 | 1-4 wt% solution, 5-30 wt% particle-forming |
| 20000 | 40 | 1-30 | 1-2 wt% solution, 3-30 wt% particle-forming |
| | 20 | 1-30 | 1-3 wt% solution, 4-30 wt% particle-forming |
| | 10 | 1-30 | 1-30 wt% solution |

### 1-4. Preparation of fibroin nano/microspheres

The following polyethylene glycol solutions were prepared separately: 40wt% solution of polyethylene glycol having a molecular weight of 2000; 40wt% solution of polyethylene glycol having a molecular weight of 4000; 40wt% solution of polyethylene glycol having a molecular weight of 6000; 40wt% solution of polyethylene glycol having a molecular weight of 10000; and 40wt% solution of polyethylene glycol having a molecular weight of 20000.

The 30wt% fibroin solution prepared in 1-1 was diluted to 8wt%, and the diluted solution was added to equal volume of polyethylene glycol solutions with concentration and molecular weight described above;
The polyethylene glycol-fibroin blending solution was incubated at room temperature for 12 h;
After incubation, the centrifuge tubes were placed in a centrifugal machine and centrifuged at room temperature, 12000 rpm/min for 10 min;
Deionized water was added for washing, the supernatant was removed, and the centrifugation and washing operations were repeated three times;
Suspension of fibroin nano/microspheres was prepared using deionized water and stored in a fridge at 4 °C, or stored after vacuum freeze-drying.

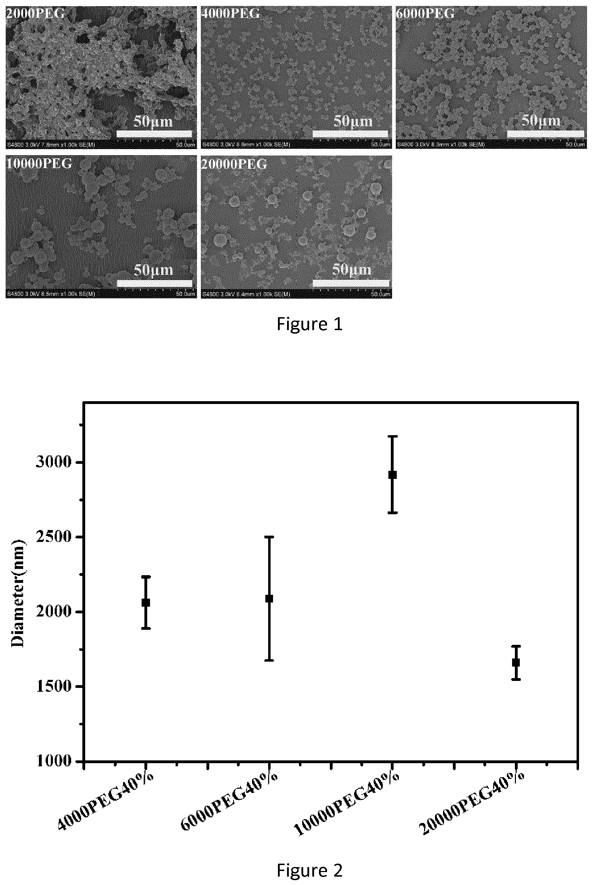

It can be seen from Figure 1 that, the fibroin nano/microspheres obtained by blending 40wt% solution of polyethylene glycol having a molecular weight of 10000 with 8wt% fibroin solution have a larger particle size than other PEGs at the same concentration.

### 1-5. Determination of particle size of fibroin nano/microspheres

Suspensions of fibroin nano/microspheres prepared in 1-4 were respectively taken out and transferred to disposable cuvettes;
The particle size of fibroin nano/microspheres in each group was measured using laser particle size analyzer in triplicate, and the average value was calculated.

As can be seen from Figure 2, which is the particle size distribution pattern of fibroin nano/microspheres, the fibroin nano/microspheres obtained by blending 40wt% solution of polyethylene glycol having a molecular weight of 10000 with 8wt% fibroin solution have a larger particle size than other PEGs at the same concentration.

### 1-6. Preparation of fibroin nano/microspheres

1. 30% solution of polyethylene glycol having a molecular weight of 4000 was prepared (indicating the molecular weight of polyethylene glycol is 4000, and the concentration is 30 wt%);
2. Fibroin solution at a concentration of 5% was prepared according to the method of Example 1.
3. Fibroin solution was mixed with the solution of polyethylene glycol at a volume ratio of 2:1, and the mixture was incubated for 24 h at a temperature of 25 °C, 45 °C and 60 °C, respectively;
4. After completion of incubation, the centrifuge tubes were placed in centrifugal machine and centrifuged at room temperature, 12000 rpm/min for 10 min;
5. Deionized water was added for washing, the supernatant was removed, and the centrifugation and washing operations were repeated three times;
6. Suspension of fibroin nano/microparticles was prepared and stored in a fridge at 4 °C, or stored after vacuum freeze-drying.

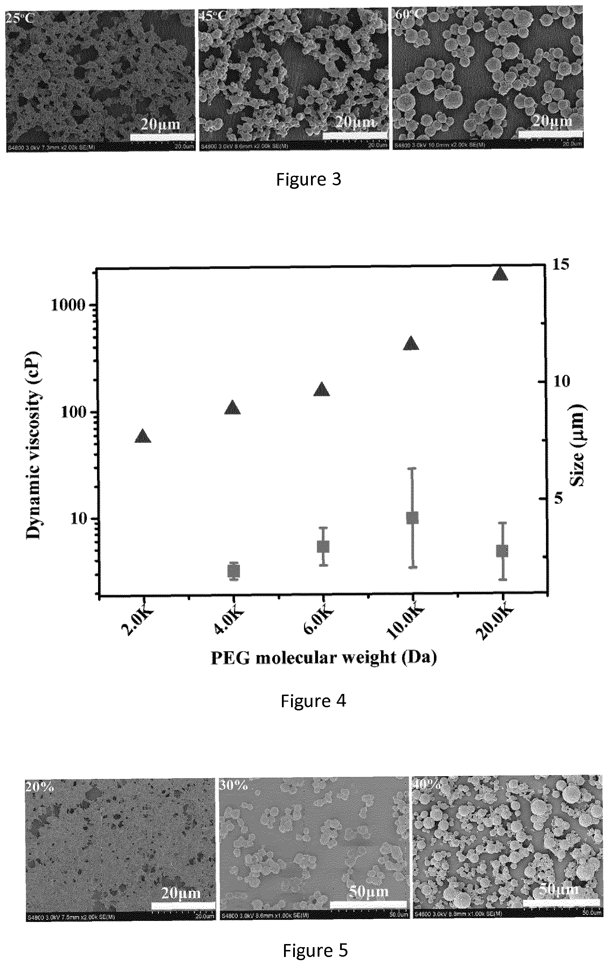

With reference to Figure 3, which is the scanning electron micrograph of fibroin nano/microparticles provided in this example via incubation at a temperature of 25 °C, 45 °C and 60 °C, respectively, it can be seen that the morphology of fibroin nano/microparticles is affected by incubation temperatures.

### Example 2

1. The polyethylene glycol solutions with different molecular weight and same concentration in examples 1-4 are selected;
2. The viscosity of above polyethylene glycol solutions was respectively measured using rheometer;
3. Fibroin solution at a concentration of 8% was prepared according to the method of Example 1;
4. Fibroin solution was mixed with the solution of polyethylene glycol at a volume ratio of 1:1, the mixture was gently agitated to blend uniformly, and then incubated for 12 h at room temperature;
5. After completion of incubation, the centrifuge tubes were placed in centrifugal machine and centrifuged at room temperature, 12000 rpm/min for 10 min;
6. Deionized water was added for washing, the supernatant was removed, and the centrifugation and washing operations were repeated three times;
7. Suspension of fibroin nano/microparticles was prepared and stored in a fridge at 4 °C, or stored after vacuum freeze-drying.

With reference to Figure 4, which is a chart of the particle size of fibroin nano/microparticles provided in this example *vs*. the viscosity of polyethylene glycol solution, in which square icon represents the particle size, and triangle icon represents viscosity, it can be seen that the particle size of fibroin nano/microparticles is affected by viscosity of polyethylene glycol solution.

### Example 3

1. 50% solution of polyethylene glycol having a molecular weight of 10000 was prepared (indicating the molecular weight of polyethylene glycol is 10000, and the concentration is 50 wt%) and then diluted to 20% and 30%, respectively;
2. The viscosity of above polyethylene glycol solutions at different concentrations was respectively measured using rheometer;
3. Fibroin solution at a concentration of 8% was prepared according to the method of Example 1;
4. Fibroin solution was mixed with the solution of polyethylene glycol at a volume ratio of 1:1, and the mixture was incubated for 24 h at room temperature;
5. After completion of incubation, the centrifuge tubes were placed in centrifugal machine and centrifuged at room temperature, 12000 rpm/min for 10 min;
6. Deionized water was added for washing, the supernatant was removed, and the centrifugation and washing operations were repeated three times;
7. Suspension of fibroin nano/microparticles was prepared and stored in a fridge at 4 °C, or stored after vacuum freeze-drying.

With reference to Figure 5, which is the scanning electron micrograph of fibroin nano/microparticles provided in this example, it can be seen that the morphology of fibroin nano/microparticles is affected by the concentration of polyethylene glycol solutions.

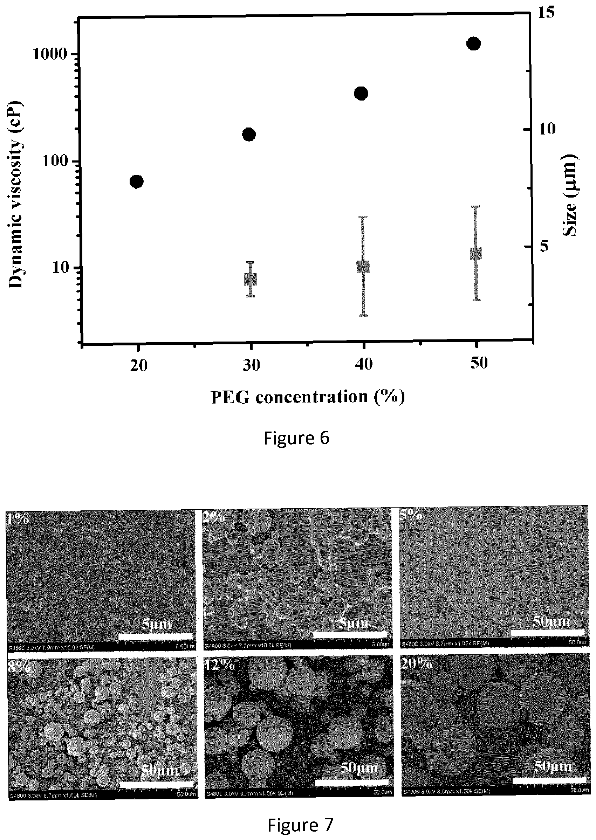

With reference to Figure 6, which is a chart of the particle size distribution of fibroin nano/microparticles provided in this example *vs*. the viscosity of polyethylene glycol solution, in which square icon represents the particle size, and triangle icon represents viscosity, it can be seen that the particle size of fibroin nano/microparticles is affected by viscosity of polyethylene glycol solution.

### Example 4

1. 50wt% solution of polyethylene glycol having a molecular weight of 10000 was prepared;
2. Fibroin solutions at concentrations of 1%, 2%, 5%, 8%, 12%, and 20wt% were prepared respectively according to the method of Example 1,
3. Fibroin solution was mixed with the solution of polyethylene glycol at a volume ratio of 1:1, and the mixture was incubated for 2 h at room temperature;
4. After completion of incubation, the centrifuge tubes were placed in centrifugal machine and centrifuged at room temperature, 12000 rpm/min for 10 min;
5. Deionized water was added for washing, the supernatant was removed, and the centrifugation and washing operations were repeated three times;
6. Suspension of fibroin nano/microparticles was prepared and stored in a fridge at 4 °C, or stored after vacuum freeze-drying.

With reference to Figure 7, which is the scanning electron micrograph of fibroin nano/microparticles provided in this example, it can be seen that the morphology of fibroin nano/microparticles is affected by the concentration of fibroin solutions.

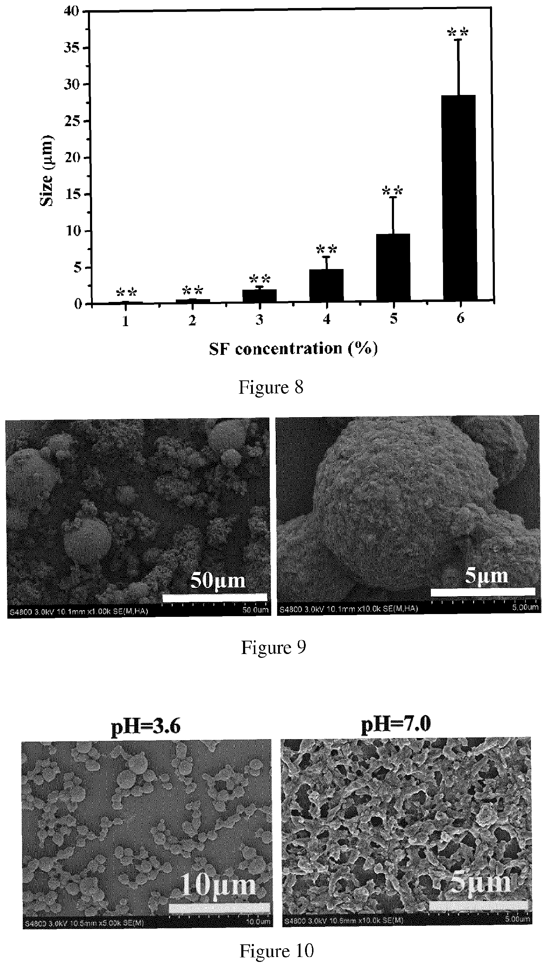

With reference to Figure 8, which is the particle size distribution pattern of fibroin nano/microparticles provided in this example, it can be seen that the particle size of fibroin nano/microparticles is affected by the concentration of fibroin solutions.

### Example 5

1. 50wt% solution of polyethylene glycol having a molecular weight of 10000 was prepared;
2. Fibroin solution at concentration of 8wt% was prepared according to the method of Example 1;
3. Fibroin solution was mixed with the solution of polyethylene glycol at a volume ratio of 10/10/1 (SF/polyethylene glycol/methanol solution), and the mixture was incubated for 2 h at room temperature;
4. After completion of incubation, the centrifuge tubes were placed in centrifugal machine and centrifuged at room temperature, 12000 rpm/min for 10 min;
5. Deionized water was added for washing, the supernatant was removed, and the centrifugation and washing operations were repeated three times;
6. Suspension of fibroin nano/microspheres was prepared and stored in a fridge at 4 °C, or stored after vacuum freeze-drying.

With reference to Figure 9, which is the scanning electron micrograph of fibroin nano/microspheres provided in this example, it can be seen that the morphology of fibroin nano/microspheres is affected by addition of methanol solution.

### Example 6

1. 60% solution of polyethylene glycol having a molecular weight of 4000 was prepared (indicating the molecular weight of polyethylene glycol is 4000, and the concentration is 60 wt%);
2. Fibroin solutions at concentration of 1% and 5% were prepared respectively;
3. The pH value of above fibroin solutions was adjusted to 3.6 and 7.0 respectively;
4. Fibroin solution was mixed with the solution of polyethylene glycol at a volume ratio of 1/1, and the mixture was incubated for 24 h at room temperature;
5. After completion of incubation, the centrifuge tubes were placed in centrifugal machine and centrifuged at room temperature, 12000 rpm/min for 10 min;
6. Deionized water was added for washing, the supernatant was removed, and the centrifugation and washing operations were repeated three times;
7. Suspension of fibroin nano/microparticles was prepared and stored in a fridge at 4 °C, or stored after vacuum freeze-drying.

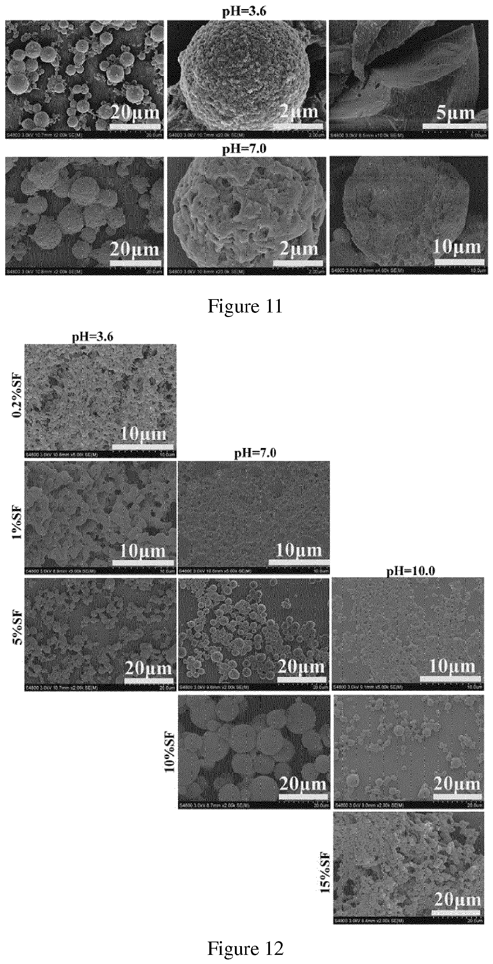

With reference to Figures 10 and 11, which are scanning electron micrographs of fibroin nano/microparticles provided in this example, with pH at 3.6 and 7.0, respectively, it can be seen that the morphology of fibroin nano/microparticles is affected by pH values of fibroin solutions.

### Example 7

1. 50% solution of polyethylene glycol having a molecular weight of 10000 was prepared (indicating the molecular weight of polyethylene glycol is 10000, and the concentration is 50 wt%);
2. Fibroin solutions at concentration of 0.2%, 1%, 5%, 10% and 15% were prepared, respectively;
3. The pH value of above fibroin solutions was adjusted to 3.6, 7.0 and 10.0, respectively;
4. Fibroin solution was mixed with the solution of polyethylene glycol at a volume ratio of 1:1, and the mixture was incubated for 24 h at room temperature;
5. After completion of incubation, the centrifuge tubes were placed in centrifugal machine and centrifuged at room temperature, 12000 rpm/min for 10 min;
6. Deionized water was added for washing, the supernatant was removed, and the centrifugation and washing operations were repeated three times;
7. Suspension of fibroin nano/microparticles was prepared and stored in a fridge at 4 °C, or stored after vacuum freeze-drying.

With reference to Figure 12, which is the scanning electron micrograph of fibroin nano/microparticles provided in this example, wherein SF at concentrations of 0.2 wt%, 1 wt%, 5 wt%, 10 wt% and 15 wt% and pH values of 3.6, 7.0 and 10.0 were blended with 50wt% solution of polyethylene glycol having a molecular weight of 10000, it can be seen that the morphology of fibroin nano/microparticles is affected by pH values of fibroin solutions.

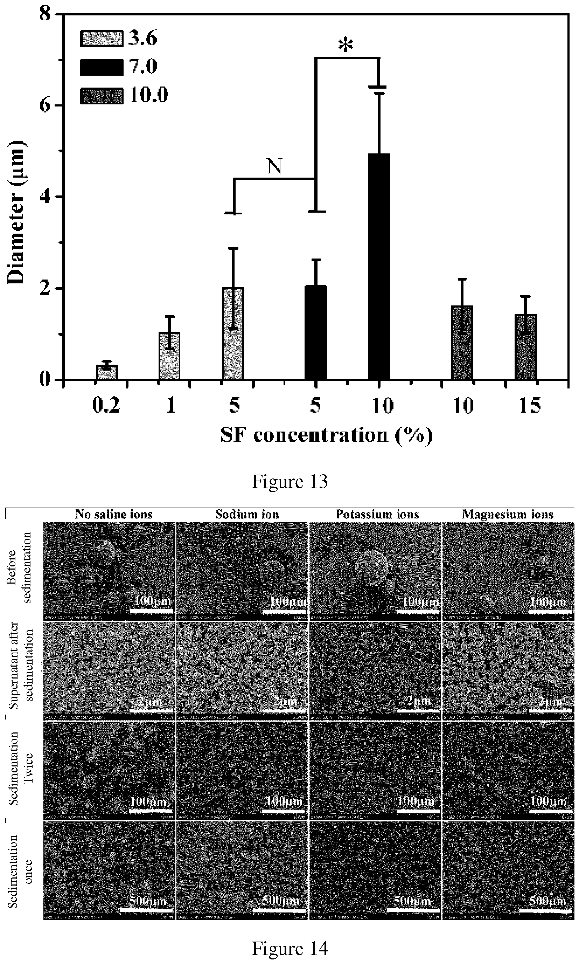

With reference to Figure 13, which is the particle size distribution pattern of fibroin nano/microparticles provided in this example, wherein SF at concentrations of 0.2 wt%, 1 wt%, 5 wt%, 10 wt% and 15 wt% and pH values of 3.6, 7.0 and 10.0 were blended with 50wt% solution of PEG having a molecular weight of 10000, it can be seen that the particle size of fibroin nano/microparticles is affected by pH values and concentrations of fibroin solutions.

### Example 8

1. 20% solution of polyethylene glycol having a molecular weight of 10000 was prepared (indicating the molecular weight of polyethylene glycol is 10000, and the concentration is 20 wt%);
2. Solutions of sodium chloride, potassium chloride and magnesium chloride at a concentration of 1 mol/L were prepared, respectively;
3. Lyophilized powder was dissolved in deionized water, and the final concentration was 30%;
4. Equal volume of above salt ion solutions was added so that the final concentration of fibroin solution is 15%, and the final concentration of salt ions is 0.5 M;
5. Fibroin/salt ions solution was mixed with the solution of polyethylene glycol at a volume ratio of 1:1, and the mixture was shaken gently to mix uniformly;
6. Absolute ethanol solution (V_{above blending solution}/Vₑₜₕₐₙₒₗ = 1/4) was added quickly, and after uniform mixing, the mixture was rapidly added to a culture dish and then transferred to an oven at 60 °C;
7. After drying, the culture dish was taken out, to which deionized water was added to make suspension;
8. The suspension was sonicated for 2 min using ultrasonic cell disruptor, with 30% amplitude of vibration;
9. Centrifugating: room temperature, 20 min, 12000 rpm;
10. Deionized water was added for washing, the supernatant was removed, and the centrifugation and washing operations were repeated three times;
11. Suspension of fibroin nano/microparticles was prepared and stored in a fridge at 4 °C, or stored after vacuum freeze-drying.

With reference to Figure 14, which is the scanning electron micrograph of fibroin nano/microparticles provided in this example, and wherein the first line is the electron micrograph before particle sedimentation, the second line is the electron micrograph of supernatants after particle sedimentation, the third line is the electron micrograph after particle sedimentationtwice, and the fourth line is the electron micrograph after particle sedimentationonce, all under treatment conditions of without ions, in the presence of sodium ion, potassium ion, and magnesium ion, respectively, it can be seen that the morphology of fibroin nano/microparticles is affected by addition of salt ions.

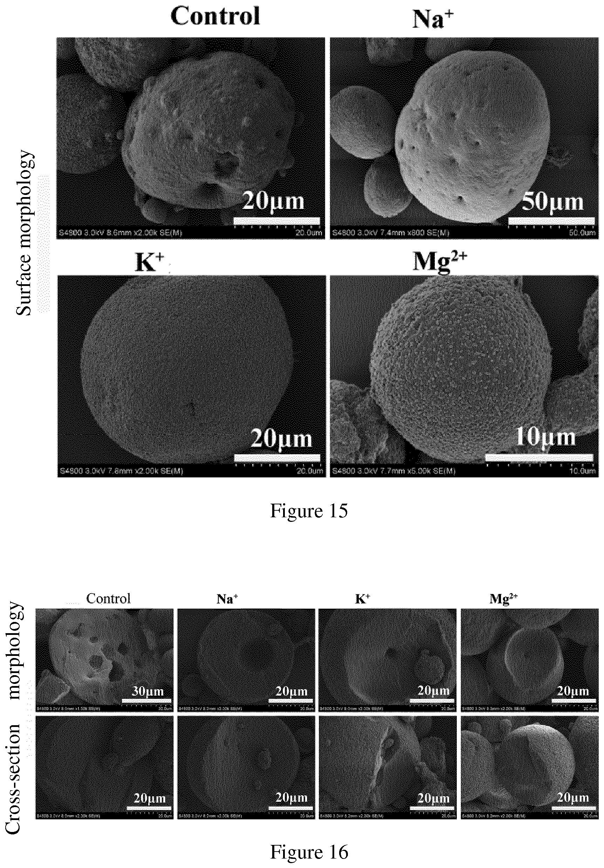

With reference to Figure 15, which is the surface morphology of single microsphere of fibroin provided in this example, it can be seen that the surface morphology of fibroin nano/microspheres is affected by addition of salt ions.

With reference to Figure 16, which is the cross section morphology of single microsphere of fibroin provided in this example, it can be seen that the cross section morphology of fibroin nano/microspheres is affected by addition of salt ions.

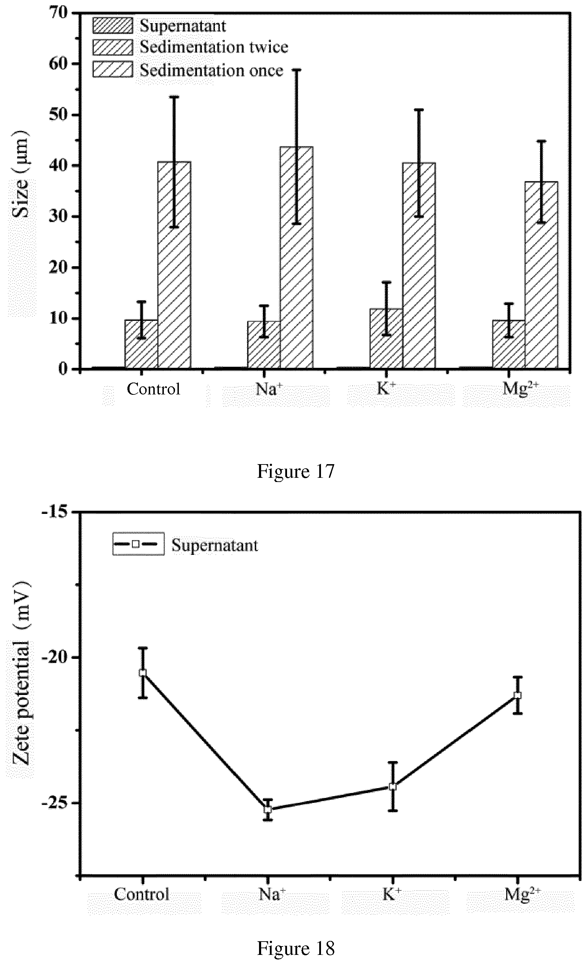

With reference to Figure 17, which is the particle size distribution pattern of fibroin nano/microparticles provided in this example, it can be seen that the average particle size of fibroin nanoparticles (supernatant) is about 350 nm, the average particle size of fibroin microparticles (sedimentation twice) is about 10 µm, and the average particle size of fibroin microparticles (sedimentation once) is about 40 µm.

With reference to Figure 18, which is the zeta potential diagram of fibroin nanoparticles provided in this example, it can be seen that the zeta potential of fibroin nanoparticles is affected by treatment with different salt ions.

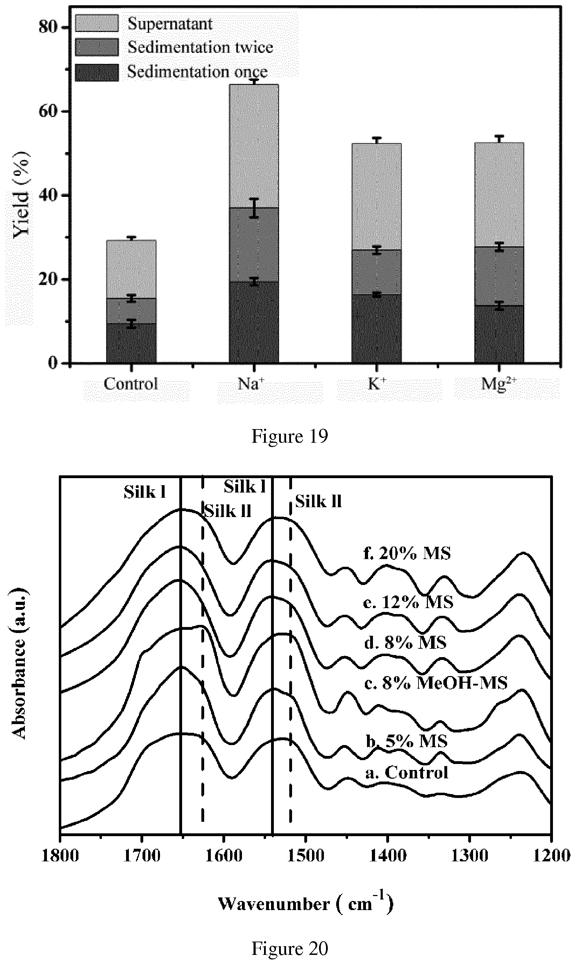

With reference to Figure 19, which is the graph of yield of fibroin nano/microparticles provided in this example, it can be seen that the yield of fibroin nano/microparticles is affected by treatment with different salt ions.

### Example 9

1. Weighing 0.1 mg of dry fibroin nano/microparticles obtained after freeze-drying in examples 4 and 5, respectively;
2. Taking small amount of potassium bromide powder and mixing with above fibroin nano/microparticles uniformly, grinding evenly with a pestle, and then tabletting with a tablet press;
3. Measuring with Fourier infrared spectrometer under the following conditions: surface total reflection, wave number range: 400-4000 cm⁻¹, and thereby, the infrared structure of each sample was tested;
4. Taking fibroin nano/microparticles prepared from 8% fibroin solution (with or without addition of methanol) and pure lyophilized fibroin powder (without adding polyethylene glycol and methanol, as control), and the secondary structure content of each sample was subjected to simulation analysis using Peakfit software.

With reference to Figure 20, which is the IR spectra of fibroin nano/microparticles provided in this example, IR spectra of fibroin nano/microparticles (5%, 8%, 12%, 20% (wt) SF blending with 50% (wt) solution of polyethylene glycol having a molecular weight of 10000; 8% (wt) blending with 50% (wt) solution of polyethylene glycol having a molecular weight of 10000 and methanol, the control), it can be seen that the secondary structure of fibroin microspheres is affected by addition of polyethylene glycol and methanol.

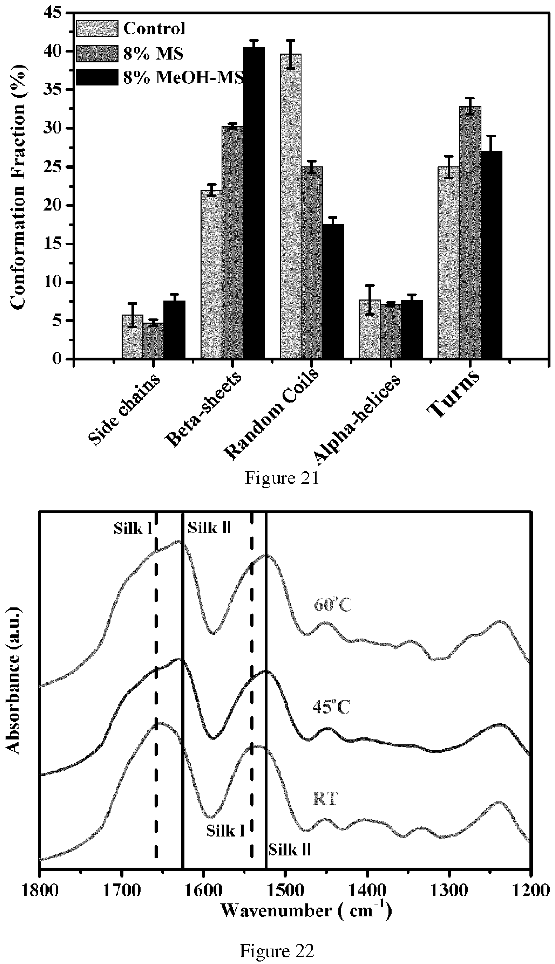

With reference to Figure 21, which is the graph of secondary structure content of fibroin nano/microparticles provided in this example as well as the control sample, it can be seen that the secondary structure content of fibroin nano/microparticles is affected by addition of polyethylene glycol and methanol.

### Example 10

1. Weighing 0.1 mg of dry fibroin nano/microparticles obtained after freeze-drying in examples 1-6, respectively;
2. Taking small amount of potassium bromide powder and mixing with above fibroin nano/microparticles uniformly, grinding evenly with a pestle, and then tabletting with a tablet press;
3. Measuring with Fourier infrared spectrometer under the following conditions: surface total reflection, wave number range: 400-4000 cm⁻¹, and thereby, the infrared structure of each sample was tested;
4. Choosing IR spectra of above fibroin nano/microparticles, and the secondary structure content of each sample was subjected to simulation analysis using Peakfit software.

With reference to Figure 22, which is the IR spectra of fibroin nano/microparticles provided in this example, it can be seen that the secondary structure of fibroin microspheres is affected by incubation temperature.

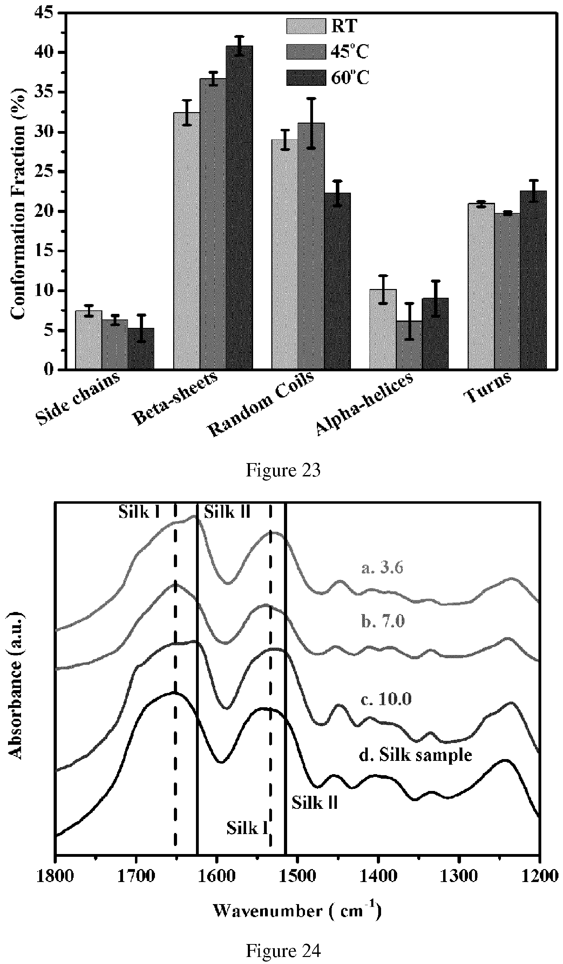

With reference to Figure 23, which is the graph of secondary structure content of fibroin nano/microparticles provided in this example as well as the control sample, it can be seen that the secondary structure content of fibroin nano/microparticles is affected by incubation temperature.

### Example 11

1. Weighing 0.1 mg of dry fibroin nano/microparticles obtained after freeze-drying in example 8;
2. Taking small amount of potassium bromide powder and mixing with above fibroin nano/microparticles uniformly, grinding evenly with a pestle, and then tabletting with a tablet press;
3. Measuring with Fourier infrared spectrometer under the following conditions: surface total reflection, wave number range: 400-4000 cm⁻¹, and thereby, the infrared structure of each sample was tested;
4. Choosing IR spectra of above fibroin nano/microparticles, and the secondary structure content of each sample was subjected to simulation analysis using Peakfit software.

With reference to Figure 24, which is the IR spectra of fibroin nano/microparticles provided in this example, it can be seen that the secondary structure of fibroin nano/microparticles is affected by pH value of fibroin solution.

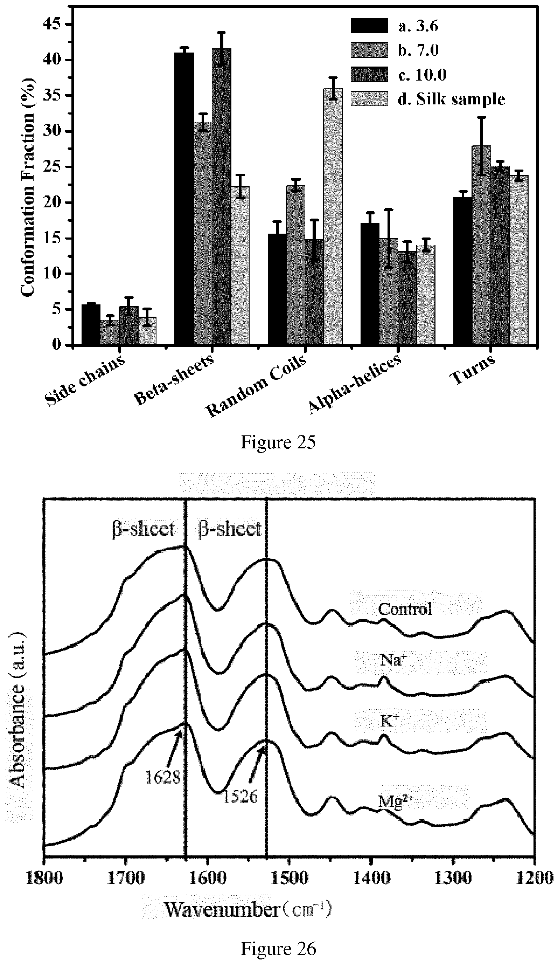

With reference to Figure 25, which is the graph of secondary structure content of fibroin nano/microparticles provided in this example as well as the control sample, it can be seen that the secondary structure content of fibroin nano/microparticles is affected by pH value of fibroin solution.

### Example 12

1. Weighing 0.1 mg of dry fibroin nano/microparticles obtained after freeze-drying in example 9;
2. Taking small amount of potassium bromide powder and mixing with above fibroin nano/microparticles uniformly, grinding evenly with a pestle, and then tabletting with a tablet press;
3. Measuring with Fourier infrared spectrometer under the following conditions: surface total reflection, wave number range: 400-4000 cm⁻¹, and thereby, the infrared structure of each sample was tested;

With reference to Figure 26, which is the IR spectra of fibroin nanoparticles (supernatant) provided in this example, it can be seen that the secondary structure of fibroin nano/microparticles is affected by treatment with salt ions.

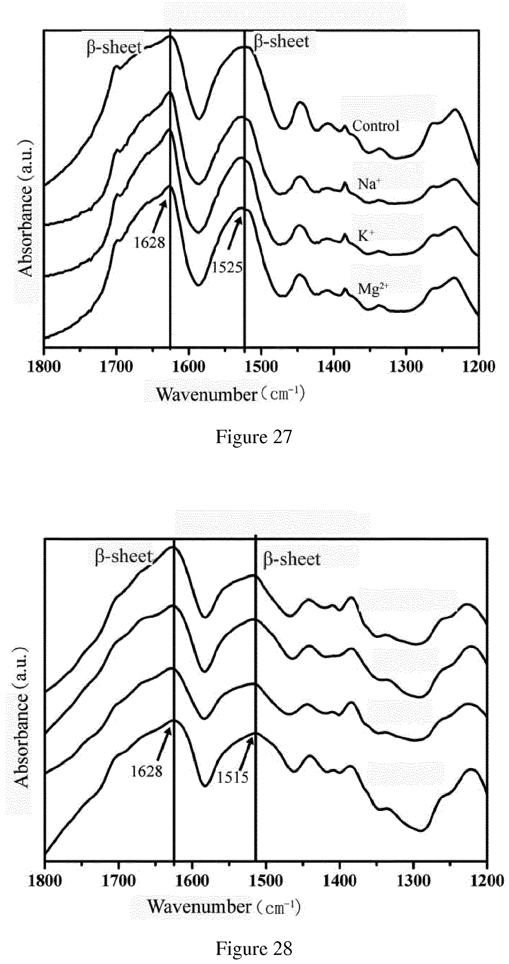

With reference to Figure 27, which is the IR spectra of fibroin microparticles (standing twice) provided in this example, it can be seen that the secondary structure of fibroin nano/microparticles is affected by treatment with salt ions.

With reference to Figure 28, which is the IR spectra of fibroin microparticles (standing once) provided in this example, it can be seen that the secondary structure of fibroin nano/microparticles is affected by treatment with salt ions.

### Example 13

### Preparation of curcumin-fibroin nano/microparticles

1. Weighing 0.5 mg and 0.9 mg of curcumin, respectively;
2. Taking 50wt% solution of polyethylene glycol having a molecular weight of 10000 (1 ml each), to respectively dissolve curcumin;
3. Preparing 5 wt% and 9 wt% fibroin solutions, 1 ml each;
4. Adding the fibroin solutions to above curcumin-polyethylene glycol solutions respectively, and gently agitating up and down with pipette, to mix the blending solution uniformly;
5. The blending solution of curcumin-polyethylene glycol-fibroin was left at room temperature and incubated for 2 h;
6. After completion of incubation, the centrifuge tubes were placed in centrifugal machine and centrifuged at room temperature, 12000 rpm/min for 10 min;
7. Deionized water was added for washing, the supernatant was removed, and the centrifugation and washing operations were repeated three times;
8. Suspension of curcumin-fibroin nano/microparticles was prepared and stored after vacuum freeze-drying;
9. Respectively, 1 mg of curcumin-fibroin nano/microparticles prepared with 5 wt% and 9 wt% fibroin solutions was weighed and added to 1 ml methanol solution, three samples per group;
10. Centrifuge tubes were vortexed for 10 min, and then placed in a rotator to rotate for 2 h at 60 °C;
11. Centrifuge tubes were taken out and centrifuged at room temperature, 12000 rpm/min for 10 min;
12. The supernatant was collected, and the absorbance of curcumin was measured at 425 nm using a microplate reader, then the content of curcumin in fibroin nano/microparticles was calculated by comparing with absorbance-concentration standard curve for curcumin, thereby the drug-loading ratio was calculated;
13. The drug-loading ratio of curcumin-fibroin nano/microparticles prepared with 5% fibroin solution was 0.27% ± 0.07%; while the drug-loading ratio of curcumin-fibroin nano/microparticles prepared with 9% fibroin solution was 0.51% ± 0.15%.

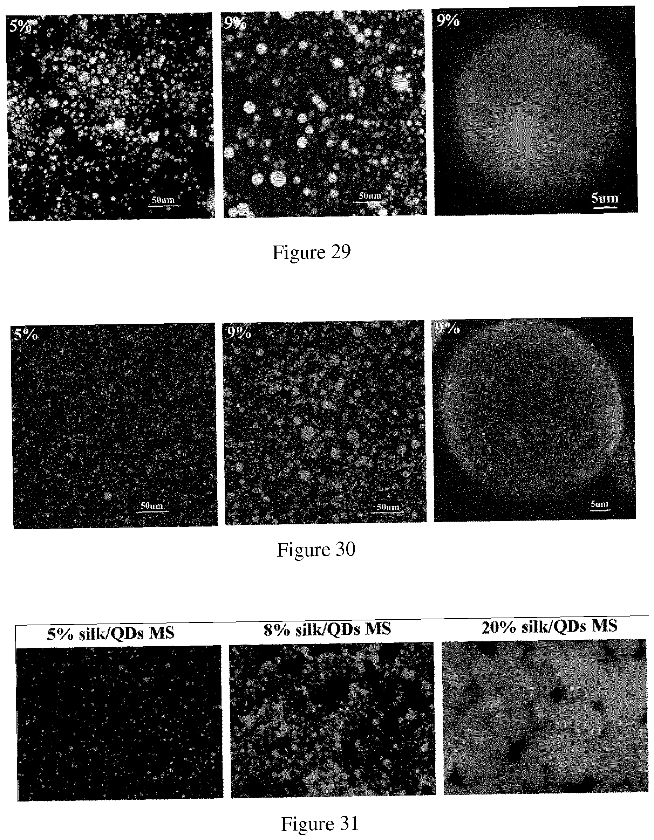

With reference to Figure 29, which is the confocal laser scanning micrograph of curcumin-fibroin nano/microparticles provided in this example, it can be seen that the distribution of curcumin in fibroin nano/microparticles is relatively uniform.

### Example 14

### Preparation of TMR-dextran-fibroin nano/microparticles

1. TMR-dextran was dissolved in water to prepare 1.0 mg/ml and 1.8 mg/ml solutions respectively;
2. 10 wt % and 18 wt% of fibroin solutions were prepared respectively;
3. The above TMR-dextran solution was mixed equal volume of fibroin solution, thereby the weight ratio of fibroin and TMR-dextran was 100/1;
4. 50wt% solution of polyethylene glycol having a molecular weight of 10000 was prepared;
5. The blending solution of fibroin and TMR-dextran was mixed with equal volume of the polyethylene glycol solution, and gently agitating up and down with pipette, to mix the blending solution uniformly;
6. The blending solution of TMR-dextran-polyethylene glycol-fibroin was left at room temperature and incubated for 2 h;
7. After completion of incubation, the centrifuge tubes were placed in a centrifugal machine and centrifuged at room temperature, 12000 rpm/min for 10 min;
8. Deionized water was added for washing, the supernatant was removed, and the centrifugation and washing operations were repeated three times;
9. Suspension of TMR-dextran-fibroin nano/microparticles was prepared and stored after vacuum freeze-drying;
10. Respectively, 1 mg of TMR-dextran-fibroin nano/microparticles prepared with 5 wt% and 9 wt% fibroin solutions was weighed and added to 1 ml lithium bromide solution, three samples per group;
11. Centrifuge tubes were vortexed for 10 min, and then placed in a rotator to rotate for 2 h at 60 °C, avoiding exposure to light;
12. Centrifuge tubes were taken out and centrifuged at room temperature, 12000 rpm/min for 10 min;
13. The supernatant was collected, and the fluorescence value of TMR-dextran was measured at excitation wavelength of 550 nm and emission wavelength of 590 nm using ELISA instrument, then the content of TMR-dextran in fibroin nano/microparticles was calculated by comparing with fluorescence value-concentration standard curve for TMR-dextran, thereby the drug-loading ratio was calculated;
14. The drug-loading ratio of TMR-dextran-fibroin nano/microparticles prepared with 5% fibroin solutions was 0.13%; while the drug-loading ratio of TMR-dextran-fibroin nano/microparticles prepared with 9% fibroin solutions was 0.26%.

With reference to Figure 30, which is the confocal laser scanning micrograph of TMR-dextran-fibroin nano/microparticles provided in this example, it can be seen that TMR-dextran mainly distributed on the surface of fibroin nano/microparticles.

### Example 15

### Preparation of CdTe-fibroin nano/microparticles

1. Solution of fibroin and CdTe quantum dots with a concentration ratio of 0.017nmol CdTe/1mg SF was prepared using deionized water, wherein the final concentration of fibroin was 5 wt%, 8 wt%, and 20 wt%;
2. 50wt% solution of polyethylene glycol having a molecular weight of 10000 was prepared;
3. The blending solution of fibroin and CdTe was mixed with equal volume of the polyethylene glycol solution, and gently agitating up and down with pipette, to mix the blending solution uniformly;
4. The blending solution of CdTe-fibroin-polyethylene glycol was left at room temperature and incubated for 2 h;
5. After completion of incubation, the centrifuge tubes were placed in centrifugal machine and centrifuged at room temperature, 12000 rpm/min for 10 min;
6. Deionized water was added for washing, the supernatant was removed and kept for testing, and then the centrifugation and washing operations were repeated three times;
7. The supernatant was collected, and the fluorescence value of CdTe quantum dots was measured at excitation wavelength of 380 nm and emission wavelength of 596 nm using a microplate reader, then the content of CdTe in supernatant was calculated by comparing with fluorescence value-concentration standard curve for CdTe, thereby the encapsulation ratio was calculated;
8. Suspension of CdTe-fibroin nano/microparticles was prepared and stored after vacuum freeze-drying;
9. The encapsulation ratio of CdTe-fibroin nano/microparticles prepared with 5% fibroin solution was 98.47% ± 0.17%; the encapsulation ratio of CdTe-fibroin nano/microparticles prepared with 8% fibroin solution was 98.89% ± 0.23%; and the encapsulation ratio of CdTe-fibroin nano/microparticles prepared with 20% fibroin solution was 96.82% ± 0.27%

With reference to Figure 31, which is the fluorescence micrograph of CdTe-fibroin nano/microparticles provided in this example, it can be seen that CdTe uniformly distributed in fibroin nano/microparticles.

### Example 16

### Preparation of curcumin-fibroin nano/microparticles

1. Weighing 1 mg of curcumin;
2. Taking 1 ml of 50wt% solution of polyethylene glycol having a molecular weight of 10000 to dissolve curcumin;
3. Preparing solution of fibroin at a concentration of 5wt% and adjusting its pH to 3.6 and 7.0;
4. Adding the fibroin solutions to above curcumin-polyethylene glycol solution respectively and gently agitating up and down with pipette, to mix the blending solution uniformly;
5. The blending solution of curcumin-polyethylene glycol-fibroin was left at room temperature and incubated for 24 h;
6. After completion of incubation, the centrifuge tubes were placed in centrifugal machine and centrifuged at room temperature, 12000 rpm/min for 10 min;
7. Deionized water was added for washing, the supernatant was removed, and the centrifugation and washing operations were repeated three times;
8. Suspension of curcumin-fibroin nano/microparticles was prepared and stored after vacuum freeze-drying;
9. Weighing 1 mg of curcumin-fibroin nano/microparticles prepared with 5% fibroin solutions at pH 3.6 and 7.0 respectively and adding to 1 ml methanol solution, three samples per group;
10. Centrifuge tubes were vortexed for 10 min, and then placed in a rotator to rotate for 2 h;
11. Centrifuge tubes were taken out and centrifuged at room temperature, 12000 rpm/min for 10 min;
12. The supernatant was collected, and the absorbance of curcumin was measured at 425 nm using a microplate reader, then the content of curcumin in fibroin nano/microparticles was calculated by comparing with absorbance-concentration standard curve for curcumin, thereby the drug-loading ratio was calculated;
13. The drug-loading ratio of curcumin-fibroin nano/microparticles prepared with 5% fibroin solution at pH 3.6 was 1.18% ± 0.08%; while the drug-loading ratio of curcumin-fibroin nano/microparticles prepared with 5% fibroin solution at pH 7.0 was 0.64% ± 0.06%.

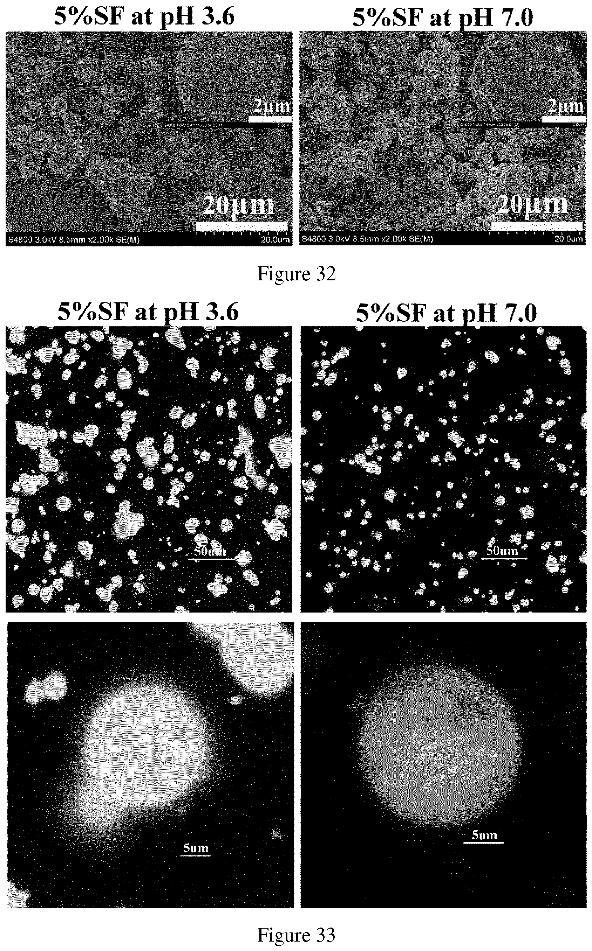

With reference to Figure 32, which is the scanning electron micrograph of curcumin-fibroin nano/microparticles provided in this example, it can be seen that the morphology of curcumin-fibroin nano/microparticles is affected by pH value of fibroin solution.

With reference to Figure 33, which is the confocal laser scanning micrograph of curcumin-fibroin nano/microparticles provided in this example, it can be seen that the distribution of curcumin in fibroin nano/microparticles is relatively uniform.

### Example 17

### Preparation of doxorubicin hydrochloride-fibroin nano/microparticles

1. Weighing 1 mg of doxorubicin hydrochloride;
2. Preparing solutions of fibroin at a concentration of 5% and pH values of 3.6 and 7.0;
3. Dissolving the doxorubicin hydrochloride particles in above fibroin solutions;
4. Preparing 50wt% solution of polyethylene glycol having a molecular weight of 10000;
5. Adding fibroin solutions containing doxorubicin hydrochloride to the polyethylene glycol solution and gently agitating up and down with pipette, to mix the blending solution uniformly;
6. The blending solution of doxorubicin hydrochloride-fibroin-polyethylene glycol was left at room temperature and incubated for 24 h;
7. After completion of incubation, the centrifuge tubes were placed in centrifugal machine and centrifuged at room temperature, 12000 rpm/min for 10 min;
8. Deionized water was added for washing, the supernatant was removed, and the centrifugation and washing operations were repeated three times;
9. Suspension of doxorubicin hydrochloride-fibroin nano/microparticles was prepared and stored after vacuum freeze-drying;
10. Weighing 1 mg of doxorubicin hydrochloride-fibroin nano/microparticles prepared with 5% fibroin solutions at pH 3.6 and 7.0 respectively and adding to 1 ml lithium bromide solution, three samples per group;
11. Centrifuge tubes were vortexed for 10 min, and then placed in a rotator to rotate for 2 h at 60 °C;
12. Centrifuge tubes were taken out and centrifuged at room temperature, 12000 rpm/min for 10 min;
13. The supernatant was collected, and the absorbance of doxorubicin hydrochloride was measured at 600 nm using a microplate reader, then the content of doxorubicin hydrochloride in fibroin nano/microparticles was calculated by comparing with absorbance-concentration standard curve for doxorubicin hydrochloride, thereby the drug-loading ratio was calculated;
14. The drug-loading ratio of doxorubicin hydrochloride-fibroin nano/microparticles prepared with 5% fibroin solution at pH 3.6 was 0.80% ± 0.06%; while the drug-loading ratio of doxorubicin hydrochloride-fibroin nano/microparticles prepared with 5% fibroin solution at pH 7.0 was 1.90% ± 0.05%.

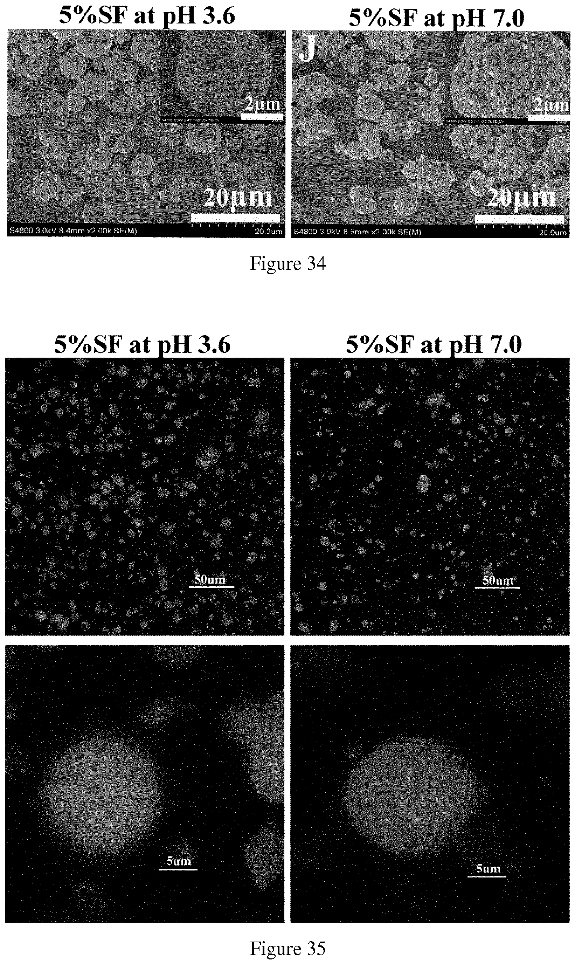

With reference to Figure 34, which is the scanning electron micrograph of doxorubicin hydrochloride-fibroin nano/microparticles provided in this example, it can be seen that the morphology of doxorubicin hydrochloride-fibroin nano/microparticles is affected by pH value of fibroin solution.

With reference to Figure 35, which is the confocal laser scanning micrograph of doxorubicin hydrochloride-fibroin nano/microparticles provided in this example, it can be seen that the distribution of doxorubicin hydrochloride in fibroin nano/microparticles is relatively uniform.

### Example 18

1. Weighing 10 mg of curcumin-fibroin nano/microparticles prepared in example 13 and adding to centrifuge tubes;
2. Phosphate buffer with a molar concentration of 0.01 mol/L (PBS, pH = 7.4) was prepared, and then 5% Tween-80 and 3% methanol were added to prepare releasing solution;
3. Adding 1 ml of above releasing solution to each sample respectively;
4. The mixture was placed in an oven at 37 °C and allowed to release drugs under shaking, and was taken out at indicated time;
5. Centrifuging at room temperature, 12000 rpm for 10 min;
6. The supernatant was collected, and the absorbance of curcumin was measured at 425 nm using a microplate reader, then the release amount of curcumin from fibroin nano/microparticles was calculated by comparing with absorbance-concentration standard curve for curcumin, thereby the cumulative release ratio was calculated.

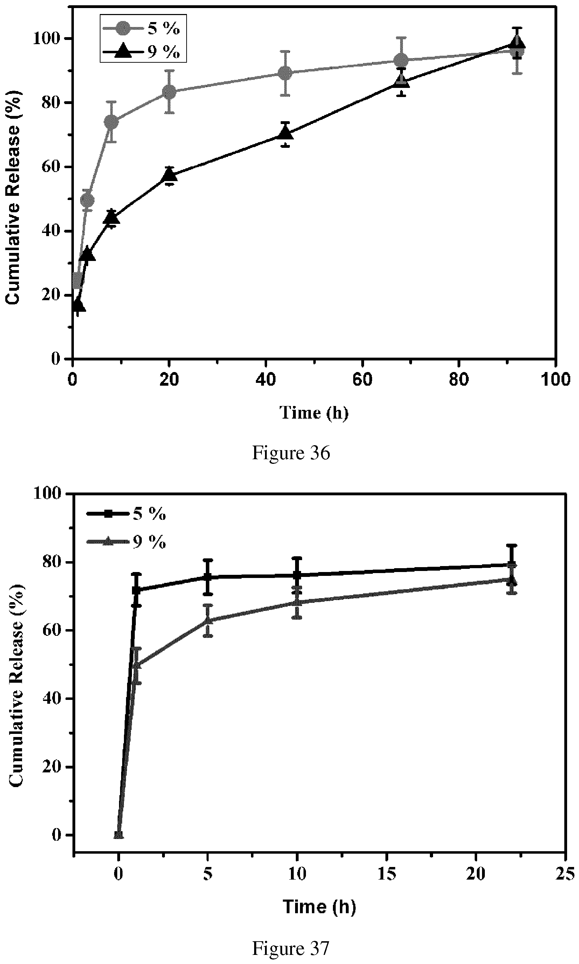

With reference to Figure 36, which is the cumulative release ratio chart of curcumin-fibroin nano/microparticles provided in this example, it can be seen that the release rate of curcumin is obviously different among fibroin-curcumin microspheres prepared at different concentrations.

### Example 19

1. Weighing 10 mg of TMR-dextran-fibroin nano/microparticles prepared in example 14 and adding to centrifuge tubes;
2. Phosphate buffer with a molar concentration of 0.01 mol/L (PBS, pH = 7.4) was prepared;
3. Adding 1 ml of PBS releasing solution to each sample respectively;
4. The mixture was placed in an oven at 37 °C and allowed to release drugs under shaking, and was taken out at indicated time;
5. Centrifuging at room temperature, 12000 rpm for 10 min;
6. The supernatant was collected, and the fluorescence value of TMR-dextran was measured at excitation wavelength of 555 nm and emission wavelength of 590 nm using a microplate reader, then the release amount of TMR-dextran from fibroin nano/microparticles was calculated by comparing with fluorescence value-concentration standard curve for TMR-dextran, thereby the cumulative release ratio was calculated.

With reference to Figure 37, which is the cumulative release ratio chart of TMR-dextran-fibroin nano/microparticles provided in this example, it can be seen that the release rate of TMR-dextran is obviously different among TMR-dextran-fibroin nano/ microspheres prepared at different concentrations.

### Example 20

1. Weighing 10 mg of CdTe-fibroin nano/microparticles prepared in example 15 and adding to centrifuge tubes;
2. Adding 1 ml of deionized water to each sample respectively;
3. The mixture was placed in an oven at 37 °C and allowed to release drugs under shaking, and was taken out at indicated time;
4. Centrifuging at room temperature, 12000 rpm for 10 min;
5. The supernatant was collected, and the fluorescence value of CdTe quantum dots was measured at excitation wavelength of 380 nm and emission wavelength of 596 nm using a microplate reader, then the release amount of CdTe quantum dots from fibroin nano/microparticles was calculated by comparing with fluorescence value-concentration standard curve for CdTe quantum dots, thereby the cumulative release ratio was calculated.

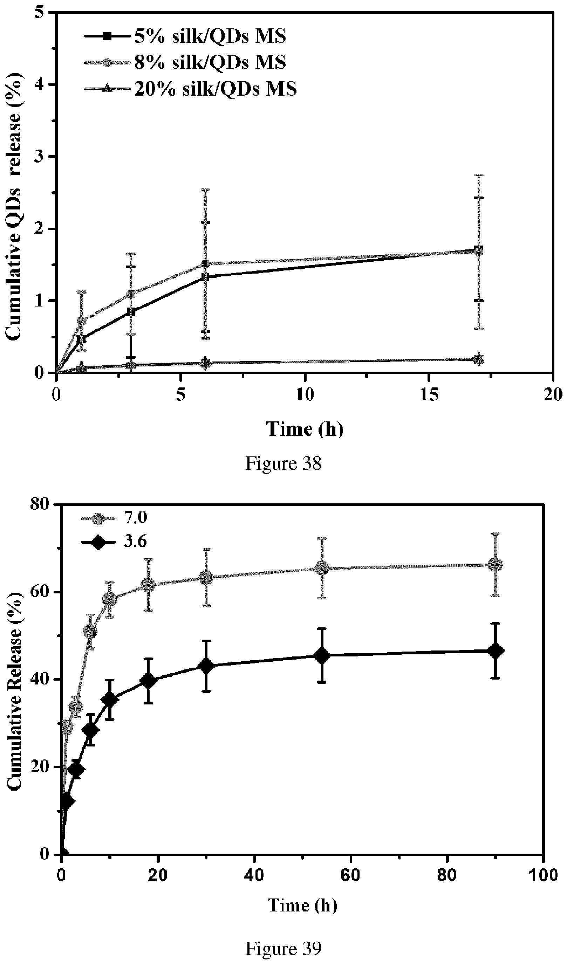

With reference to Figure 38, which is the cumulative release ratio graph of CdTe-fibroin nano/microparticles provided in this example, it can be seen that the release rate of CdTe quantum dots is obviously different among CdTe-fibroin nano/microparticles prepared at different concentrations.

### Example 21

1. Weighing 10 mg of curcumin-fibroin nano/microparticles prepared in example 16 and adding to centrifuge tubes;
2. Phosphate buffer with a molar concentration of 0.01 mol/L (PBS, pH = 7.4) was prepared, and then 5% Tween-80 and 3% methanol were added to prepare releasing solution;
3. Adding 1 ml of above releasing solution to each sample respectively;
4. The mixture was placed in an oven at 37 °C and allowed to release drugs under shaking, and was taken out at indicated time;
5. Centrifuging at room temperature, 12000 rpm for 10 min;
6. The supernatant was collected, and the absorbance of curcumin was measured at 425 nm using a microplate reader, then the release amount of curcumin from fibroin nano/microparticles was calculated by comparing with absorbance-concentration standard curve for curcumin, thereby the cumulative release ratio was calculated.

With reference to Figure 39, which is the cumulative release ratio graph of curcumin-fibroin nano/microparticles provided in this example, it can be seen that the release rate of curcumin is obviously different among fibroin-curcumin microspheres prepared at different concentrations.

### Example 22

1. Weighing 10 mg of doxorubicin hydrochloride-fibroin nano/microparticles prepared in example 17 and adding to centrifuge tubes;
2. Phosphate buffer with a molar concentration of 0.01 mol/L (PBS, pH = 4.0) was prepared;
3. Adding 1 ml of PBS releasing solution to each sample respectively;
4. The mixture was placed in an oven at 37 °C and allowed to release drugs under shaking, and was taken out at indicated time;
5. Centrifuging at room temperature, 12000 rpm for 10 min;
6. The supernatant was collected, and the absorbance of doxorubicin hydrochloride was measured at 480 nm using a microplate reader, then the release amount of doxorubicin hydrochloride from fibroin nano/microparticles was calculated by comparing with absorbance-concentration standard curve for doxorubicin hydrochloride, thereby the cumulative release ratio was calculated.

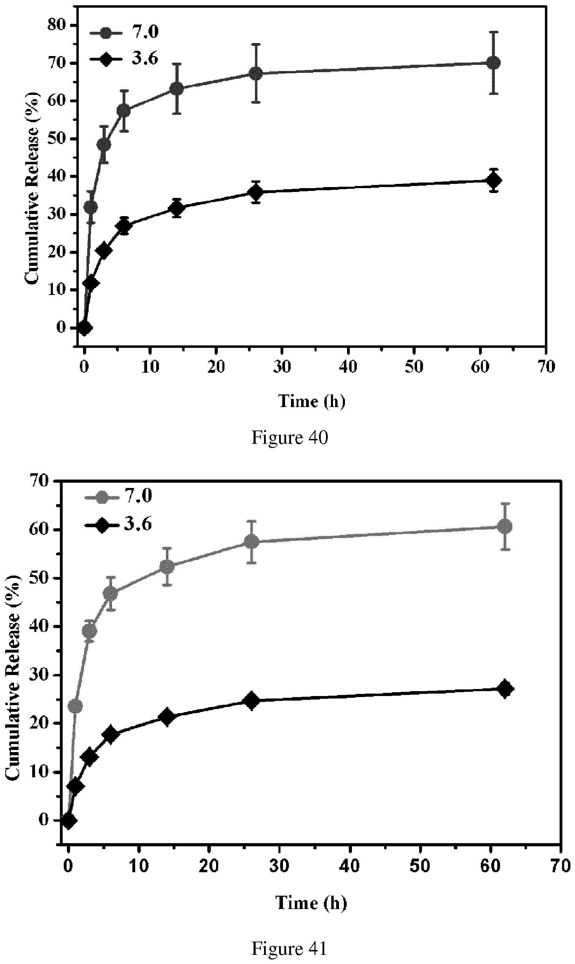

With reference to Figure 40, which is the cumulative release ratio graph of doxorubicin hydrochloride-fibroin nano/microparticles provided in this example, it can be seen that the release rate of doxorubicin hydrochloride is obviously different among fibroin-doxorubicin hydrochloride microspheres prepared at different concentrations.

### Example 23

1. Weighing 10 mg of doxorubicin hydrochloride-fibroin nano/microparticles prepared in example 17 and adding to centrifuge tubes;
2. Phosphate buffer with a molar concentration of 0.01 mol/L (PBS, pH = 7.4) was prepared;
3. Adding 1 ml of PBS releasing solution to each sample respectively;
4. The mixture was placed in an oven at 37 °C and allowed to release drugs under shaking, and was taken out at indicated time;
5. Centrifugating at room temperature, 12000 rpm for 10 min;
6. The supernatant was collected, and the absorbance of doxorubicin hydrochloride was measured at 480 nm using a microplate reader, then the release amount of doxorubicin hydrochloride from fibroin nano/microparticles was calculated by comparing with absorbance-concentration standard curve for doxorubicin hydrochloride, thereby the cumulative release ratio was calculated.

With reference to Figure 41, which is the cumulative release ratio graph of doxorubicin hydrochloride-fibroin nano/microparticles provided in this example, it can be seen that the release rate of doxorubicin hydrochloride is obviously different among doxorubicin hydrochloride-fibroin nano/microspheres prepared at different concentrations.

### Example 24

### Preparation of octreotide-fibroin nano/microparticles

1. Preparing 20 mg/ml octreotide solution with deionized water;
2. Dissolving lyophilized fibroin powder in deionized water, and the concentration was 100 mg/ml;
3. Blending the above solutions (2 ml each), thereby the weight ratio of fibroin to octreotide was 5/1;
4. 4 ml of 50wt% solution of polyethylene glycol having a molecular weight of 10000 was taken out;
5. The above three solutions were mixed together and incubated at room temperature for 24 h;
6. After completion of incubation, the centrifuge tubes were placed in centrifugal machine and centrifuged at room temperature, 12000 rpm/min for 10 min;
7. The supernatant was reserved, and the centrifugation and washing operations were repeated three times, then was suspended and freeze-dried;

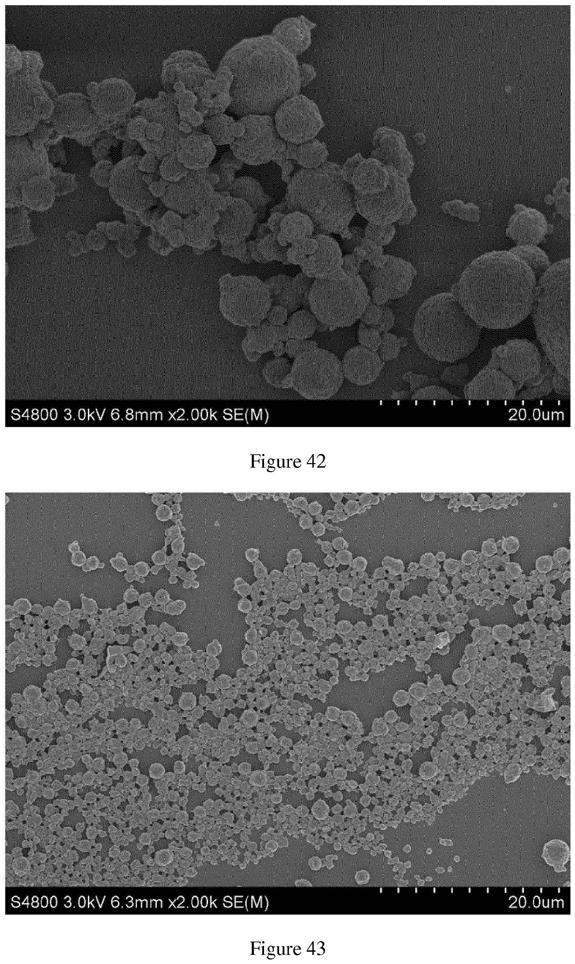

See Figure 42, which is the scanning electron micrograph of nano/microparticles of octreotide-fibroin provided in this example.

### Example 25

### Preparation of TMR-bovine serum albumin-fibroin nano/microparticles

1. TMR-bovine serum albumin solution (1 ml) at a concentration of 0.5 mg/ml was prepared with deionized water;
2. Lyophilized fibroin powder was dissolved with deionized water, and the concentration was 60 mg/ml, the volume was 5 ml;
3. The above solutions were mixed, wherein the weight ratio of fibroin to TMR-bovine serum albumin was 600/1;
4. 6 ml of 50% solution of polyethylene glycol having a molecular weight of 10000 was taken out;
5. The above three solutions were mixed together and incubated at room temperature for 24 h;
6. After completion of incubation, the centrifuge tubes were placed in centrifugal machine and centrifuged at room temperature, 12000 rpm/min for 10 min;
7. The supernatant was reserved, and the centrifugation and washing operations were repeated three times, then was suspended and freeze-dried;
8. The content of TMR-bovine serum albumin in supernatant was measured, the fluorescence value of TMR-bovine serum albumin was measured at excitation wavelength of 550 nm and emission wavelength of 590 nm using a microplate reader and the fluorescence-concentration standard curve was plotted, then the fluorescence of test sample was measured and the content of TMR-bovine serum albumin in supernatant was calculated, thereby the encapsulation ratio of TMR-bovine serum albumin was calculated, which was 40.4 ± 4.2%.

See Figure 43, which is the scanning electron micrograph of TMR-bovine serum albumin-fibroin nano/microparticles provided in this example.

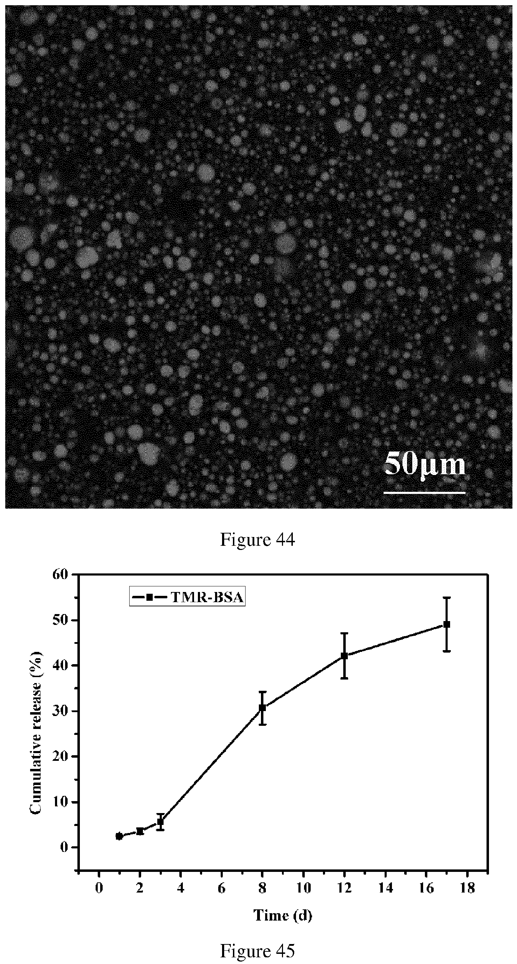

See Figure 44, which is the confocal laser scanning micrograph of TMR-bovine serum albumin-fibroin nano/microparticles provided in this example.

### Example 26

1. Weighing 5 mg of TMR-bovine serum albumin-fibroin nano/microparticles prepared in example 25;
2. Phosphate buffer with a molar concentration of 0.01 mol/L (PBS, pH = 7.4) was prepared;
3. Adding 1 ml of PBS releasing solution to each sample respectively;
4. The mixture was placed in an oven at 37 °C and allowed to release drugs under shaking, and was taken out at indicated time;
5. Centrifuging at room temperature, 12000 rpm for 10 min;
6. The supernatant was collected, and the fluorescence value of TMR-bovine serum albumin was measured at excitation wavelength of 555 nm and emission wavelength of 590 nm using a microplate reader, then the release amount of TMR-bovine serum albumin from fibroin nano/microparticles was calculated by comparing with fluorescence value-concentration standard curve for TMR-bovine serum albumin, thereby the cumulative release ratio was calculated.

With reference to Figure 45, it can be seen that TMR-bovine serum albumin in TMR-bovine serum albumin-fibroin nano/microparticles shows obvious controlled release effect.

### Example 27

1. 20% solution of polyethylene glycol having a molecular weight of 10000 was prepared (indicating the molecular weight of polyethylene glycol is 10000, and the concentration is 20 wt%)
2. Solutions of sodium chloride, potassium chloride and magnesium chloride at a concentration of 0.2 mol/L were prepared, respectively;
3. Lyophilized powder was dissolved in deionized water, and the final concentration was 30%;
4. 24 mg of doxorubicin hydrochloride was weighed and dissolved with deionized water (4 ml) at room temperature, to obtain doxorubicin hydrochloride solution at 6 mg/ml;
5. To the above doxorubicin hydrochloride solution of 6 mg/ml, equal volume of above salt ion solutions was added to obtain an doxorubicin hydrochloride solution at a final concentration of 3 mg/ml;
6. The above fibroin solution was diluted with equal volume of above doxorubicin hydrochloride solution dissolved with above salt ions solutions, to obtain a 15% fibroin solution wherein the concentration of doxorubicin was 1.5 mg/ml, the concentration of salt ions was 0.1 mol/L (the weight ratio of doxorubicin hydrochloride to fibroin was 1/100);
7. The solution of doxorubicin-fibroin, treated with salt ions, was mixed with the solution of polyethylene glycol at a volume ratio of 1/1, and then gently shaken to mix uniformly;
8. Absolute ethanol solution (V_{above blending solution}/Vₑₜₕₐₙₒₗ = 1/4) was added quickly, and after uniform mixing, the mixture was rapidly added to a culture dish and then transferred to an oven at 60 °C;
9. After drying, the culture dish was taken out, to which deionized water was added to make suspension;
10. The suspension was sonicated for 2 min using ultrasonic cell disruptor, with 30% amplitude of vibration;
11. Centrifuging: room temperature 20 min, 12000 rpm;
12. Deionized water was added for washing, the supernatant was removed, and the centrifugation and washing operations were repeated three times;
13. Suspension of doxorubicin hydrochloride-fibroin nano/microparticles was prepared and stored in a fridge at 4 °C, or stored after vacuum freeze-drying.

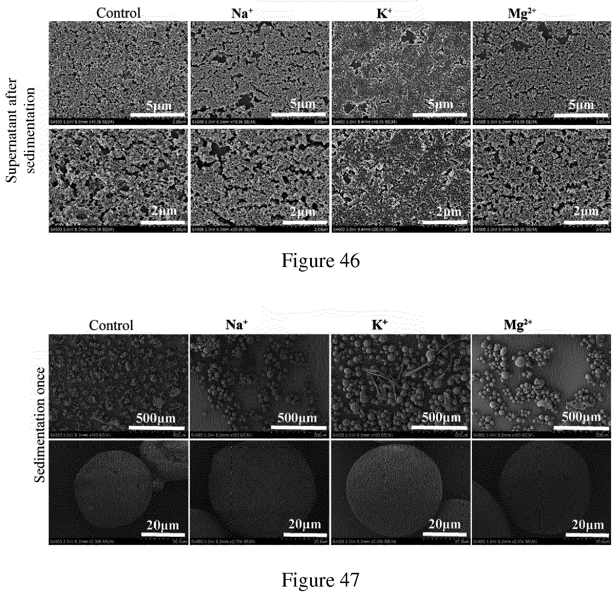

With reference to Figure 46, which is the scanning electron micrograph of doxorubicin hydrochloride-fibroin nanoparticles (supernatant) provided in this example, it can be seen that the morphology of doxorubicin hydrochloride-fibroin nanoparticles is affected by addition of salt ions.

With reference to Figure 47, which is the scanning electron micrograph of doxorubicin hydrochloride-fibroin microparticles provided in this example, it can be seen that the morphology of doxorubicin hydrochloride-fibroin microparticles (standing once) is affected by addition of salt ions.

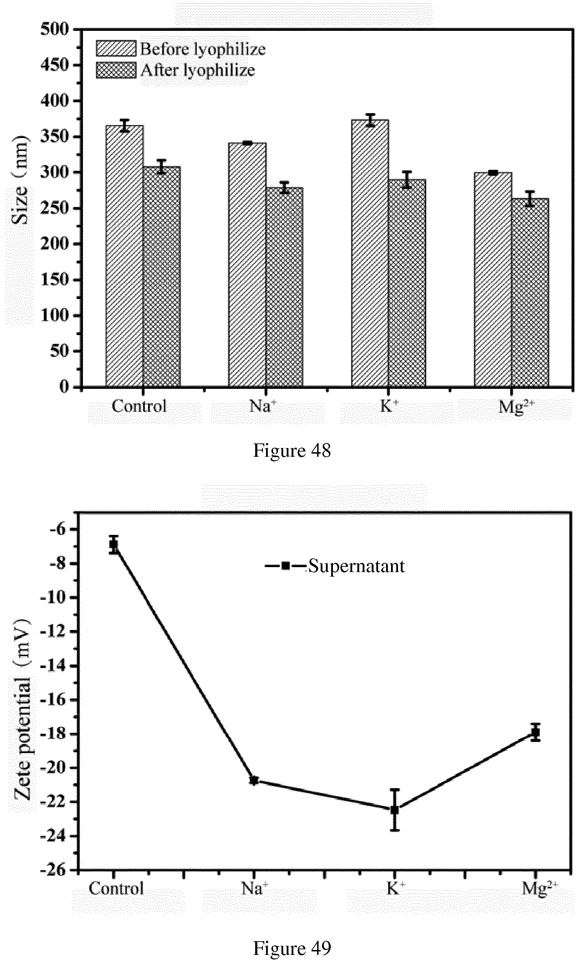

With reference to Figure 48, which is the particle size distribution pattern of doxorubicin hydrochloride-fibroin nanoparticles (supernatant) provided in this example (before freeze-drying and suspending after freeze-drying), it can be seen that the particle size of doxorubicin hydrochloride-fibroin nanoparticles is affected by addition of salt ions and ultrasonic dispersion.

With reference to Figure 49, which is the zeta potential diagram of doxorubicin hydrochloride-fibroin nanoparticles (supernatant) provided in this example, it can be seen that the zeta potential of doxorubicin hydrochloride-fibroin nanoparticles is affected by addition of salt ions.

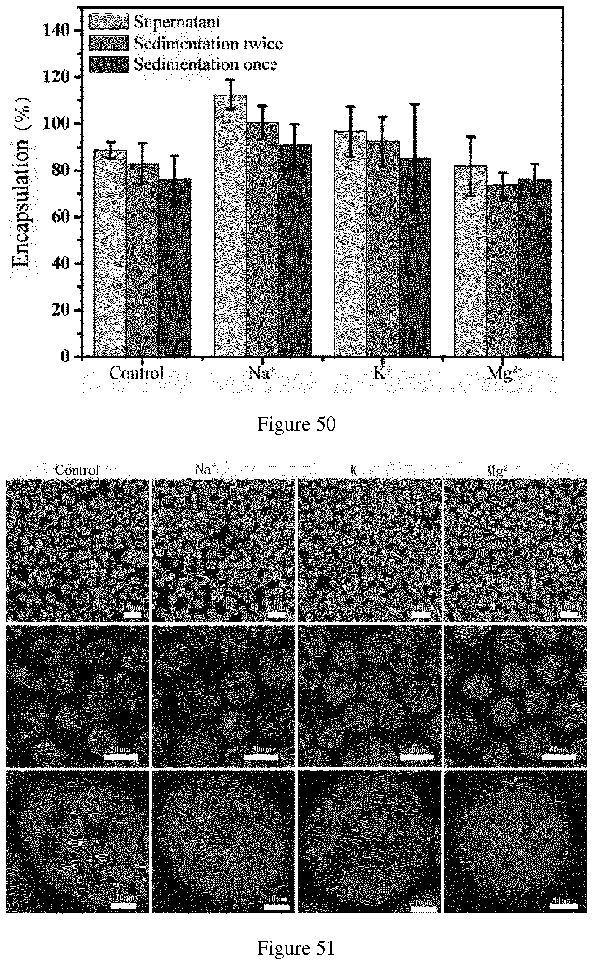

With reference to Figure 50, which is the encapsulation ratio map of doxorubicin hydrochloride-fibroin nano/microparticles provided in this example, it can be seen that the loading efficiency of doxorubicin in doxorubicin hydrochloride-fibroin nanoparticles is high, close to 100%, and the encapsulation ratio is also affected by different salt ions.

With reference to Figure 51, which is the confocal laser scanning micrograph of doxorubicin hydrochloride-fibroin microparticles (sedimentation once) provided in this example, it can be seen that the distribution of doxorubicin loaded in doxorubicin hydrochloride-fibroin microparticles is relatively uniform, and the intensity is very high.

The above are only preferred embodiments of the present invention, and the invention is not limited to these. It should be pointed out that ordinary skilled persons in the art can make various improvements and variations without departing from the technical principle of the invention, and such improvements and variations should also be regarded as under the protection of the invention.

## Claims

1. A method for preparation of fibroin nano/microspheres using polyethylene glycol, **characterized by** comprising the following steps:
1-30 wt% fibroin solution and 10-60 wt% polyethylene glycol solution are placed at a temperature of 4-60 °C for 30 min, and then the fibroin solution and the polyethylene glycol solution are uniformly mixed at a certain ratio, and after incubation for a certain period of time, the mixture is subjected to centrifugal washing to afford suspension of fibroin nano/microspheres.

2. Use of the method for preparation of fibroin nano/microspheres using polyethylene glycol in the controlled release of drugs, **characterized by** comprising the following steps:
dissolving the drug in 10-60 wt% polyethylene glycol solution or dissolving the drug in 1-30 wt% fibroin solution, and then the fibroin solution and the polyethylene glycol solution are uniformly mixed to produce a blending solution, and after incubation the blending solution for a certain period of time and centrifugal washing, drug-loaded fibroin nano/microspheres used for controlled release of drugs are obtained.

3. The method according to claim 2, **characterized in that** when the drugs are hydrophobic, said hydrophobic drugs are dissolved in the solution of polyethylene glycol before mixing with the fibroin solution to prepare the blending solution; and for other drugs than hydrophobic drugs, said other drugs are firstly dissolved in the solution of fibroin before mixing with the solution of polyethylene glycol to prepare the blending solution.

4. The method according to claim 3, **characterized in that** said hydrophobic drugs include curcumin, while said other drugs include hydrophilic drug adriamycin, polypeptide drug octreotide, protein drug bovine serum albumin, macromolecular hydrophilic model drug glucan or fluorescent labeled CdTe quantum dot.

5. The method according to claim 2, **characterized in that** the mass ratio of fibroin contained in said fibroin solution to said drug is in the range of 1000/1-1/10.

6. The method according to claim 1 or 2, **characterized in that** the molecular weight of polyethylene glycol is in the range of 2000-20000.

7. The method according to claim 6, **characterized in that** when the molecular weight of polyethylene glycol is 4000, 6000, its mass percent is 30-60%; when the molecular weight of polyethylene glycol is 10000, its mass percent is 20-50%; when the molecular weight of polyethylene glycol is 20000, its mass percent is 20-40%.

8. The method according to claim 1 or 2, **characterized in that** the pH value of said fibroin solution is in the range of 3.0-11.0.

9. The method according to claim 1 or 2, **characterized in that** the salt ions concentration for treatment of fibroin solution is in the range of 1 mol/L to 0.01 mol/L.

10. The method according to claim 1 or 2, **characterized in that** the volume ratio of fibroin solution to polyethylene glycol solution is in the range of 5:1-1:10.

11. The method according to claim 1 or 2, **characterized in that** said blending solution is incubated at a temperature from room temperature to 60 °C for 0.5-24 h.

12. The method according to claim 1 or 2, **characterized in that** the dilution range of said fibroin solution is 5-20 wt%.

13. The method according to claim 1 or 2, **characterized in that** the preparing procedure of said fibroin solution is as follows: the degummed, boiled-off silk is soaked in a 9.3 M solution of LiBr and placed in an oven at 60 °C for 4 h, and after dissolving, the fibroin solution is obtained; said fibroin solution is poured into a dialysis bag and dialyzed against deionized water for 3 days; after completion of dialysis, the fibroin solution is centrifuged to remove insoluble impurities, and then poured into a dialysis bag and dialyzed against 15 wt% solution of polyethylene glycol having a molecular weight of 20000 for 24 h, to obtain the concentrated solution of fibroin.
